(19) Europäisches Patentamt
European Patent Office
Office européen des brevets

(11) EP 2 189 442 B1

(12) **EUROPEAN PATENT SPECIFICATION**

(45) Date of publication and mention of the grant of the patent:
**01.10.2014 Bulletin 2014/40**

(21) Application number: **09176568.5**

(22) Date of filing: **19.11.2009**

(51) Int Cl.:
***C07C 231/12*** (2006.01) ***C07C 237/22*** (2006.01)
***C07C 51/083*** (2006.01) ***C07C 59/90*** (2006.01)
***C07C 259/06*** (2006.01) ***C07D 313/00*** (2006.01)
***C07D 321/00*** (2006.01) ***C07D 307/33*** (2006.01)

(54) **Process and intermediates for the preparation of aliskiren**

Verfahren und Zwischenprodukte für die Herstellung von Aliskiren

Procédé et intermédiaires pour la préparation d'aliskiren

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **16.01.2009 EP 09150808**
**20.11.2008 PCT/EP2008/065919**

(43) Date of publication of application:
**26.05.2010 Bulletin 2010/21**

(73) Proprietor: **Krka Tovarna Zdravil, D.D., Novo Mesto**
**8501 Novo Mesto (SI)**

(72) Inventors:
- **Kidemet, Davor**
**42000, Varazdin (HR)**
- **Zupet, Rok**
**1211, Ljubljana (SI)**
- **Smodis, Janez**
**8323, Ursna sela (SI)**
- **Stefane, Bogdan**
**2317, Oplotnica (SI)**
- **Pozgan, Franc**
**2277, Sredisce ob Dravi (SI)**

(74) Representative: **UEXKÜLL & STOLBERG**
**Patentanwälte**
**Beselerstrasse 4**
**22607 Hamburg (DE)**

(56) References cited:
**WO-A1-02/02508**
**WO-A1-2007/006532**
**WO-A1-2007/048620**
**WO-A1-2008/119804**
**WO-A2-2007/045420**
**GB-A- 2 431 648**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 189 442 B1

## Description

The present invention relates to a process and intermediates for the manufacture of a compound having the following formula (I):

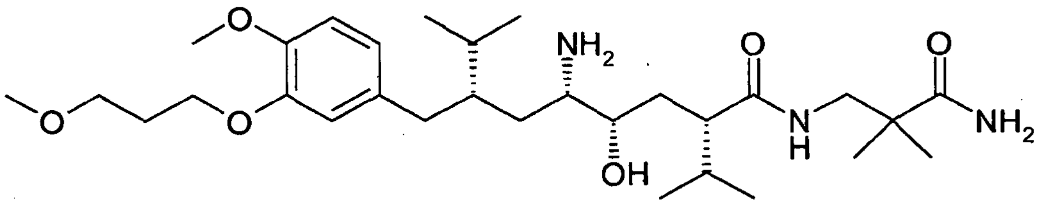

(I)

i.e. (2S,4S,5S,7S)-5-amino-N-(2-carbamoyl-2-methylpropyl)-4-hydroxy-2-isopropyl-7-[4-methoxy-3-(3-methoxypropoxy)benzyl]-8-methylnonanamide (hereinafter referred to by its generic name "aliskiren"), or pharmaceutically acceptable salts thereof.

Aliskiren is an orally effective renin inhibitor. It is useful in the treatment of hypertension and its hemifumarate salt is commercially available under the trade names Tekturna® and Rasilez®.

The synthesis of 2(S), 4(S), 5(S), 7(S)-2,7-dialkyl-4-hydoxy-5-amino-8-aryl-octanoyl amides such as aliskiren is known from EP-A-0 678 503. Further multistage syntheses of substituted octanoyl amides are for example described in EP-A-1 215 201, WO-A-01/09079, WO-A-01/09083, WO-A-02/02508, WO-A-02/08172, WO-A-03/103653, WO-A-2006/024501, WO-A-2006/131304, WO-A-2007/039183, GB-A-2 431 640, GB-A-2 431 641, GB-A-2 431 642, GB-A-2 431 643, GB-A-2 431 644, GB-A-2 431 645, GB-A-2 431 646, GB-A-2 431 647, GB-A-2 431 648, GB-A-2 431 649, GB-A-2 431 650, GB-A-2 431 651, GB-A-2 431 652, GB-A-2 431 653 and GB-A-2 431 654. WO 2007/048620 describes the synthesis of octenoic acid derivatives.

Nevertheless, there is still a need for a process for preparing aliskiren which process is characterized in that aliskiren is obtainable in a high total yield and in a high degree of diastereomeric and enantiomeric purity.

Surprisingly, it has now been found that aliskiren is obtainable by a process as depicted in Scheme 1 below.

Scheme 1:

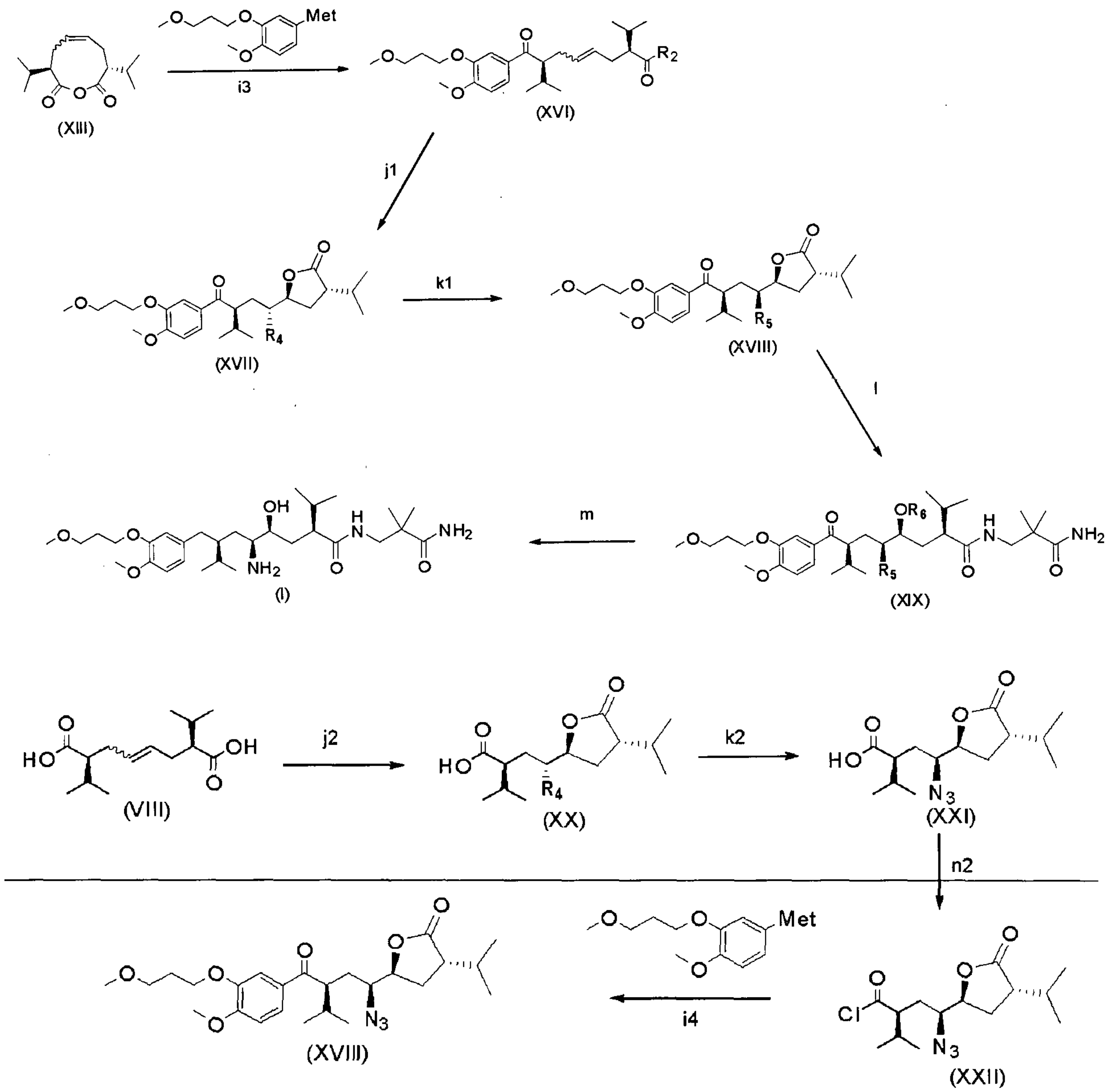


(XIII) (XVI) (XVII) (XVIII) (I) (XIX) (VIII) (XX) (XXI) (XVIII) (XXII)

Further suitable reaction steps to obtain the intermediates depicted in Scheme 1 are shown in Schemes 2 and 3 below.

Scheme ~~3~~2:

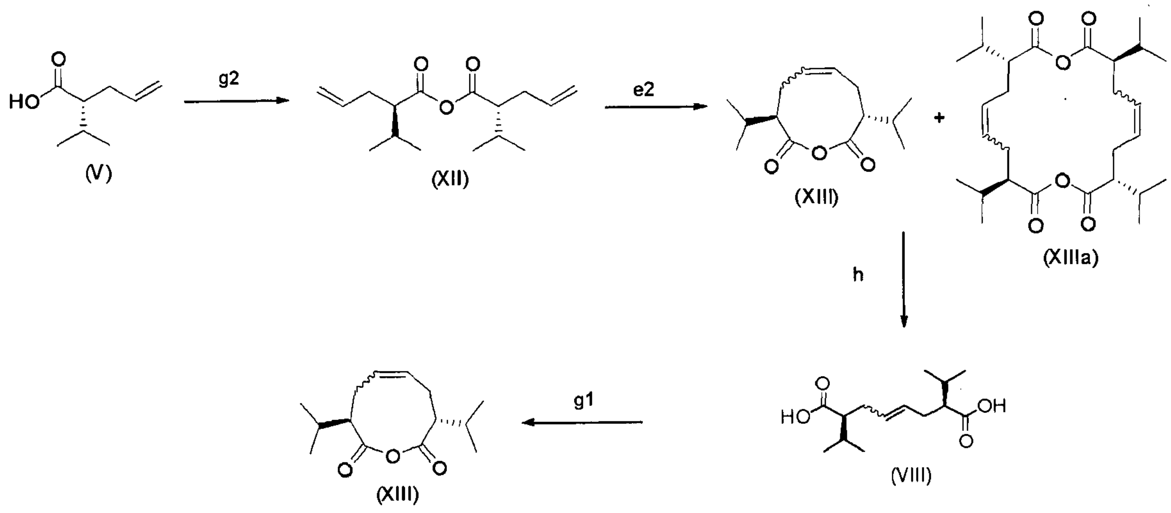


Scheme ~~4~~3:

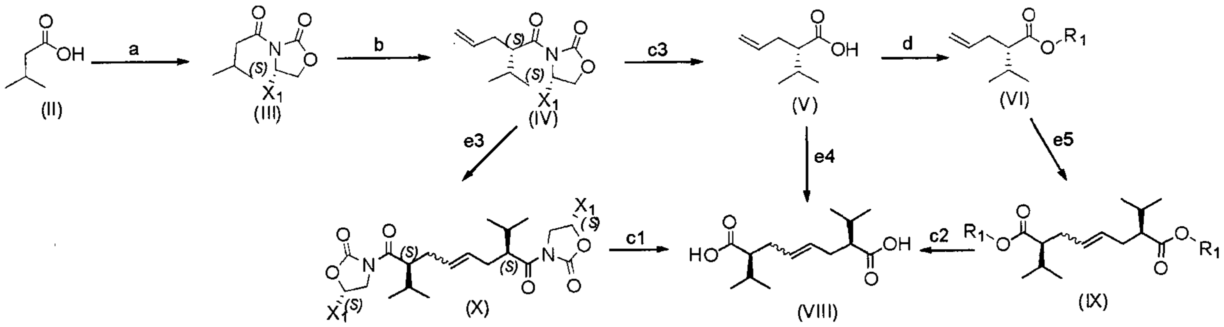


Thus, it is disclosed a process for preparing aliskiren of the following formula (I) or pharmaceutically acceptable salts thereof

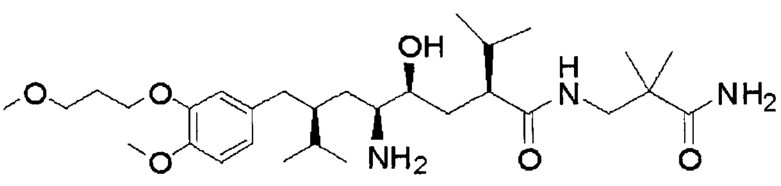

(I)

comprising

(m) converting a compound of formula (XIX)

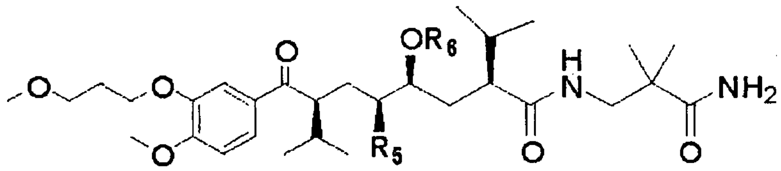

(XIX)

,

wherein

$R_5$ is $-N_3$ or $-NR_7R_8$, with $R_7$ and $R_8$ being independently selected from the group consisting of -H, alkyl, alkylaryl, i.e. an alkyl group which is substituted with an aryl group, aryloxyaryl, heterocyclyl, cycloalkyl, -C(O)-O-$R_9$ and -C(O)-$R_9$, wherein each of the alkyl, alkylaryl, aryloxyaryl, heterocyclyl and cycloalkyl can be unsubstituted or substituted, and wherein $R_9$ is selected from the group of consisting of alkyl, alkenyl, alkynyl, aryl and alkylaryl, with the proviso that if any one of $R_7$ and $R_8$ is -H, then the other of $R_7$ and $R_8$ is not -H or alkyl, or $R_7$ and $R_8$ combined together with the nitrogen atom to which they are attached form a 5 to 7 membered heterocyclic ring, using reaction conditions well known in the art; and

$R_6$ is -H or a hydroxyl protecting group,

into the compound of formula (I) or a pharmaceutically acceptable salt thereof.

Preferred embodiments of reaction step (m) of the first aspect of the invention are described below under the title "Step (m) :".

The invention relates to a process for preparing a compound of formula (XVI)

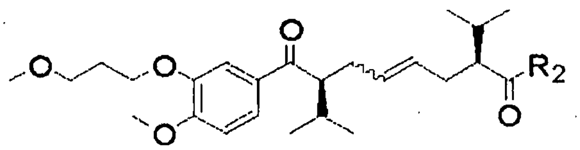

(XVI)

wherein $R_2$ is selected from the group consisting of halo, -OH, amino, alkyl, alkoxy, aryl, cycloalkyl, heterocyclyl or heteroaryl, wherein each of said amino, alkyl, alkoxy, aryl, cycloalkyl, heterocyclyl or heteroaryl can be unsubstituted or substituted,

comprising

(i3) reacting a compound of formula (XIII)

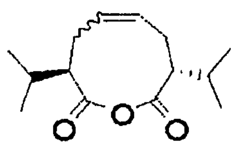

(XIII)

or a dimer thereof

with a compound of formula (XXIII)

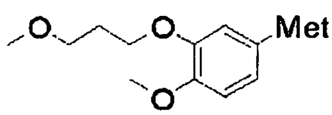

(XXIII)

wherein Met is a metal moiety,

to form a compound of formula (XVI).

Preferred embodiments of reaction step (i3) are described below under the title "step:".

Thus, the invention is also directed to a process for preparing aliskiren of the following formula (I) or pharmaceutically acceptable salts thereof

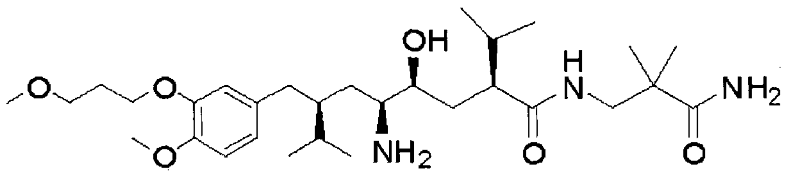

(I)

comprising
preparing a compound of formula (XVI) by a process according to step (i3) as described above and
converting the compound of formula (XVI) into a compound of formula (I) or a pharmaceutically acceptable salt thereof.

Generally, the manufacture of compound (XIII) as well as the conversion of compound (XVI) to aliskiren can be performed in any manner known in the art.

However, it is particularly preferred that the preparation of compound (XIII), which is used as starting materials in the processes according to the invention, as well as the conversion of compound (XVI) to aliskiren, which compound (XVI) is obtained as intermediate in the process according to the invention, comprises at least one of the following reaction steps.

Preferably, the compound of formula (XVI) is converted into aliskiren of formula (I) or a pharmaceutically acceptable salt thereof by converting it into a compound of formula (XIX) and carrying out step (m) as defined above to obtain a compound of formula (I) or a pharmaceutical acceptable salt thereof.

Preferably, the compound of formula (XIX) is prepared by

(1) reacting a compound of formula (XVIII)

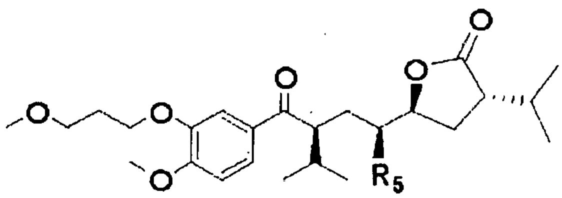

(XVIII) ,

wherein $R_5$ is defined as above,
with an amine of formula (XIV)

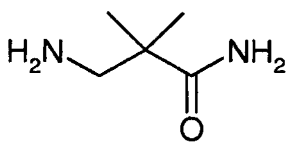

(XIV).

The compound of formula (XVIII) is preferably prepared by

(j1) lactonizing a compound of formula (XVI)

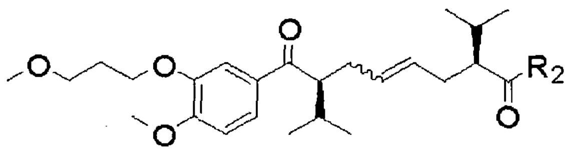

(XVI)

wherein $R_2$ is selected from the group consisting of halo, -OH, amino, alkyl, alkoxy, aryl, cycloalkyl, heterocyclyl or heteroaryl, wherein each of said amino, alkyl, alkoxy, aryl, cycloalkyl, heterocyclyl or heteroaryl can be unsubstituted or substituted,
into a compound of formula (XVII)

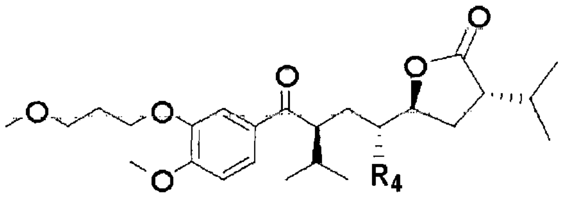

(XVII)

,

wherein $R_4$ is -Cl, -Br, -I or -OH,
and

(k1) converting the compound of formula (XVII) to form the compound of formula (XVIII).

Generally, the compound of formula (XVI) can be prepared by

(i3) reacting a compound of formula (XIII)

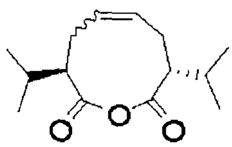

(XIII)

or a dimer thereof
with a compound of formula (XXIII)

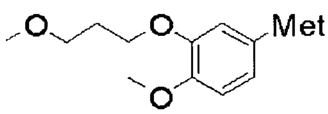

(XXIII)

wherein Met is a metal moiety,
to form the compound of formula (XVI).

Further, it is preferred that the compound of formula (XIII) is prepared by

(g1) converting a compound of formula (VIII)

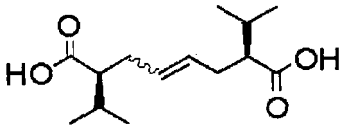

(VIII)

into the compound of formula (XIII);
or

(e2) converting a compound of formula (XII)

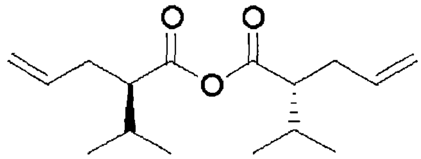

(XII)

into the compound of formula (XIII).

The intermediate compound of formula (VIII) used in the above reaction steps is usually present as a mixture of the corresponding (Z)- and (E)-isomers and is preferably prepared by

(e2) converting a compound of formula (XII)

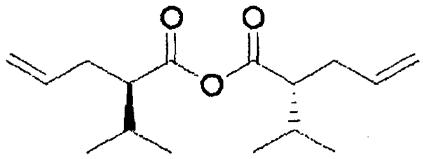

(XII)

into a mixture comprising the compounds of formula (XIII) and (XIIIa)

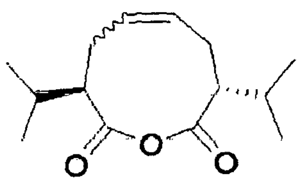

(XIII)

+

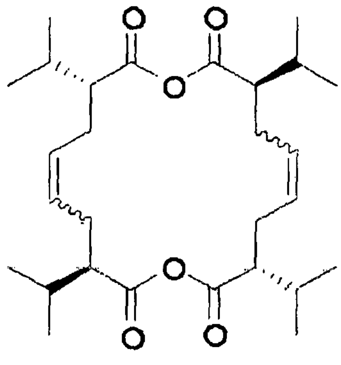

(XIIIa)

and

(h) converting the mixture comprising the compounds of formula (XIII) and (XIIIa) into the compound of formula (VIII);
or

(e3) converting a compound of formula (IV)

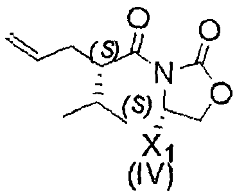

(IV)

wherein $X_1$ is an aryl or alkylaryl group, into a compound of formula (X)

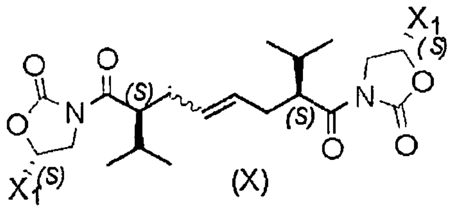

(X)

and

(c1) converting the compound of formula (X) into the compound of formula (VIII); or

(e4) converting a compound of formula (V)

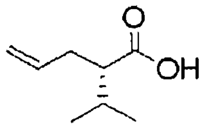

(V)

into the compound of formula (VIII); or

(d) converting a compound of formula (V)

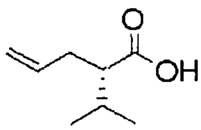

(V)

into a compound of formula (VI)

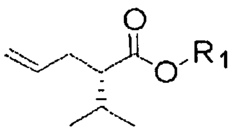

(VI)

wherein $R_1$ is an alkyl or alkylaryl group,

(e5) converting the compound of formula (VI) into a compound of formula (IX)

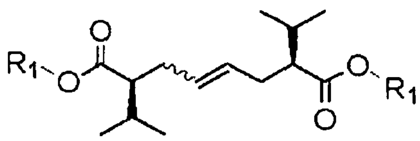

(IX)

wherein $R_1$ is an alkyl or alkylaryl group, and

(c2) converting the compound of formula (IX) into the compound of formula (VIII).

Preferably, the compound of formula (XII) is prepared by

(g2) converting a compound of formula (V)

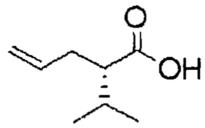

(V)

into the compound of formula (XII).

Moreover, it is preferred to prepare the compound of formula (V) by

(c3) converting a compound of formula (IV)

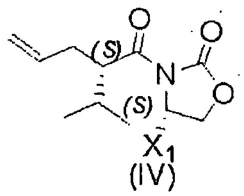

(IV)

wherein $X_1$ is an aryl or alkylaryl group,
into the compound of formula (V).

Finally, the compound of formula (IV) is preferably prepared by

(a) converting a compound of formula (II)

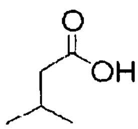

(II)

into a compound of formula (III)

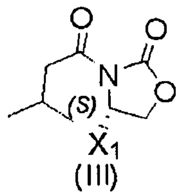

(III)

wherein $X_1$ is an aryl or alkylaryl group, and

(b) converting the compound of formula (III) into the compound of formula (IV).

The compound of formula (I) prepared by the various process variants of the invention is aliskiren in the form of its free base or a pharmaceutically acceptable salt thereof. Examples of suitable salts include addition salts with inorganic acids such as hydrochloride acid, hydrobromic acid, phosphoric acid, sulfuric acid and nitric acid, or organic acids such as carboxylic acids, e.g. fumaric acid, acetic acid, citric acid, tartaric acid, maleic acid and glutamic acid, or sulfonic acids, e.g. methanesulfonic acid. Aliskiren or the pharmaceutically acceptable salts thereof can be prepared in a non-crystalline, i.e. amorphous, form or a crystalline form including any polymorphic or pseudopolymorphic forms such as solvates like hydrates. Most preferably, the compound of formula (I) is prepared in the form of its hemifumarate salt.

Preferably, the obtained aliskiren or salt thereof has a purity of at least 99.5 %.

In addition to the compound of formula (I), also compounds of formulae (II) to (XXIII) can not only be present and used in the form as is shown by formulae (II) to (XXIII), but can also be present and used in the form of a salt thereof, e.g. a salt formed by an organic or inorganic base and an acidic part of the compound such as an alcohol group or a salt formed by an organic or inorganic acid and a basic part of the compound such as an amino group. Moreover, compounds (II) to (XXIII) can also be present and used in the form of a solvate thereof such as a solvate formed by a solvent used for the preparation of a compound of formulae (II) to (XXIII). Thus, whenever a compound is referred to in this description or the appended claims, a corresponding salt or solvate is also intended, provided such is possible or appropriate under the circumstances.

Furthermore, the compounds (II) to (XXIII) used in the process according to the invention can comprise protecting groups. Protecting groups may be introduced to protect the functional groups present from undesired reaction with reaction components under the conditions used for carrying out a particular chemical transformation of the present invention. The need and choice of protecting groups for a particular reaction is known to those skilled in the art and depends on the nature of the functional group to be protected (for example, amino, hydroxyl, thiol etc.), the structure and stability of the molecule of which the substituent is a part and the reaction conditions. Well-known protecting groups which meet these conditions as well as their introduction and removal are described, for example, in Kocienski, "Protecting Groups", Georg Thieme Verlag, Stuttgart, (2005); Greene and Wuts, "Protective Groups in Organic Synthesis", John Wiley & Sons, NY (1999).

The individual reaction steps used in the process according to the invention can generally be carried out according to standard methods, in the presence or absence of a diluent or solvent, which is preferably inert to the reagents, of catalysts and/or inert atmospheres. The temperature used in the reaction steps of the process according to the invention can generally range from low temperatures such as -78°C to room temperature or elevated temperatures, which are preferably at or near the boiling point of the solvent used. In addition, the individual reaction steps are generally carried out at atmospheric or super-atmospheric pressure.

Preferably, the individual reaction steps of the process according to the invention are carried out in the presence of a solvent. Suitable solvents are water, organic solvents and ionic liquids, especially polar organic solvents or mixtures of at least two solvents. In particular, examples of suitable solvents include hydrocarbons such as petroleum ether, pentane, hexane, heptane, cyclohexane, methylcyclohexane, benzene, toluene, xylene; halogenated hydrocarbons such as dichloromethane, chloroform, tetrachloroethane, chlorobenzene; ethers such as diethyl ether, dibutyl ether, tetrahydrofuran, 2-methyltetrahydrofuran, dioxane, ethylene glycol dimethyl, diethyl ether; carbonic esters and lactones such as methyl acetate, ethyl acetate, isopropyl acetate, methyl propionate, valerolactone; N,N-substituted carboxamides and lactams such as dimethylformamide, dimethylacetamide, N-methylpyrrolidinone; ketones such as acetone, methylisobutylketone, cyclohexanone; sulfoxides and sulfones such as dimethysulfoxide, dimethylsulfone, tetramethylene sulfone; alcohols such as methanol, ethanol, n- or i-propanol, n-, i- or t-butanol, pentanol, hexanol, cyclohexanol, cyclohexanediol, benzyl alcohol, ethylene glycol, diethylene glycol, propanediol, butanediol, ethylene glycol momomethyl or monoethyl ether, diethylene glycol monomethyl or monoethyl ether; nitriles such as acetonitrile, propionitrile; tertiary amines such as trimethylamine, triethylamine, tripropylamine, tributylamine, pyridine, N-methylpiperazine, N-methylmorpholine; organic acids such as acetic acid, formic acid; ionic liquids; and mixtures thereof as well as mixtures of at least one of the abovementioned solvents with water.

Moreover, the individual reaction steps of the process according to the invention are preferably carried out under an inert atmosphere, more preferably under nitrogen atmosphere.

The compounds of formulae (I) to (XXIII) may be isolated using conventional methods known in the art, e.g. extraction, distillation, crystallization and filtration as well as combinations thereof.

In the following, preferred embodiments of substituents $X_1$, $R_1$, $R_2$, $R_3$, $R_4$, $R_5$, $R_6$, $R_7$, $R_8$, $R_9$ and Met are given. These preferred embodiments are supposed to be applicable to all aspects of the invention and can independently be combined with each other.

$X_1$ is an aryl or alkylaryl group, i.e. an alkyl group which is substituted with an aryl group. In particular, $X_1$ is phenyl or - $(CH_{2)n}$-aryl, wherein n is preferably ranging from 1 to 6 and aryl is preferably phenyl or naphthyl. Most preferably, $X_1$ is benzyl, i.e. $-CH_2C_6H_5$, or phenyl.

Preferably, $R_1$ represents $C_1$-$C_6$ alkyl or an alkylaryl group, i.e. an alkyl group which is substituted with an aryl group, in particular $-(CH_2)_n$-aryl, wherein n is preferably ranging from 1 to 6 and aryl is preferably phenyl or naphthyl. Most preferably, $R_1$ is methyl.

Preferably, $R_2$ represents -Cl, a hydroxyl group, $C_1$-$C_6$ alkoxyl, alkylaryl, i.e. an alkyl group which is substituted with an aryl group, in particular $-(CH_2)_n$-aryl, wherein n is preferably ranging from 1 to 6 and aryl is preferably phenyl or naphthyl, (S) -4-$X_1$-oxazolidin-2-onyl such as (S)-4-benzyloxazolidin-2-onyl or (S)-4-phenyloxazolidin-2-onyl, or a N,O-dimethylhydroxylamino group.

Preferably, $R_3$ is -Cl.

Preferably, $R_4$ is -Br.

Preferably, $R_5$ is $-N_3$ or $-NR_7R_8$, wherein $R_7$ is H and $R_8$ is alkylaryl, such as benzyl, or -C(O)-O-$R_9$ with $R_9$ being selected from alkyl, such as tert.-butyl, alkenyl, such as allyl, and alkylaryl, such as benzyl or fluorenylmethyl. Most preferably, $R_5$ is $-N_3$.

Preferably, $R_6$ is -H or a hydroxyl protecting group selected from the group consisting of trimethyl silyl, triethyl silyl, tert.-butyl methyl silyl, p-methoxybenzyl, methoxymethyl, acetyl, benzoyl, tosyl and mesyl. More preferably, $R_6$ is -H.

Preferably, $R_7$ is H and $R_8$ is alkylaryl, such as benzyl, or -C(O)-O-$R_9$ with $R_9$ being selected from alkyl, such as tert.-butyl, alkenyl, such as allyl, and alkylaryl, such as benzyl or fluorenylmethyl.

Preferably, $R_9$ is alkyl, such as tert.-butyl, alkenyl, such as allyl, or alkylaryl, such as benzyl or fluorenylmethyl.

Preferably, Met represents a metal moiety selected from -MgCl, -MgBr, -MgI, -ZnCl, -ZnBr, -ZnI or -Li, and more preferably is -MgBr or -ZnCl. In formulae used in this description or the appended claims, the term " 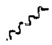 " represents a covalent bond which comprises an (E) stereoisomer as well as a (Z) stereoisomer of the respective olefin.

In the following, preferred embodiments of the individual reaction steps used in the various aspects of the invention are given. These preferred embodiments can independently be combined with each other.

Step (a):

Isovaleric acid having formula (II) is preferably reacted with a (S)-4-$X_1$-oxazolidin-2-one such as (S)-4-benzyloxazolidin-2-one or (S)-4-phenyloxazolidin-2-one to yield a compound of formula (III). It is particularly preferred that the isovaleric acid (II) is firstly converted into isovaleric acid halogenide and said isovaleric acid halogenide is then reacted with an (S)-4-$X_1$-oxazolidone-2-one anion such as (S)-4-benzyloxazolidin-2-one or (S)-4-phenyloxazolidin-2-one anion. The (S) -4-$X_1$-oxazolidone-2-one anion can be obtained by treating (S)-4-$X_1$-oxazolidone-2-one with a suitable base.

The generation of isovaleric acid halogenide such as isovaleric chloride can be performed with oxalyl chloride alone or in the presence of a base such as pyridine, in a solvent such as dimethylformamide. Further, isovaleric acid can be deprotonated with a base such as lithium hydroxide followed by treatment of the obtained carboxylate with trimethysilylchloride and oxalyl chloride. Alternatively, the generation of isovaleric acid halogenide such as isovaleric chloride can be performed using thionyl chloride alone or in the presence of a base such as lithium hydroxide, in anhydrous solvents such as toluene or benzene. Most preferably, the isovaleric acid halogenide is generated by treatment of isovaleric acid with oxalyl chloride in toluene at a temperature of 5°C to 65°C using dimethylformamide (Tetrahedron 1997, 53, 13757).

The (S)-4-$X_1$-oxazolidin-2-one anion can be generated by treating (S)-4-$X_1$-oxazolidin-2-one with a suitable base such as n-butyl litium in a solvent such as tetrahydrofuran or dimethylformamide. The temperature for this reaction is preferably about -78°C. Preferably, the obtained (S)-4-$X_1$-oxazolidin-2-one anion such as (S)-4-benzyloxazolidin-2-one or (S)-4-phenyloxazolidin-2-one anion is reacted with the isovaleric acid halogenide such as isovaleric chloride in a solvent such as tetrahydrofuran or dimethylformamide.

Step (b):

The compound of formula (III) is preferably reacted with allyl halogenide such as allyl bromide or allyl iodide to

yield a compound of formula (IV). It is particularly preferred that the compound of formula (III) is condensed with allyl bromide or allyl iodide in the presence of a suitable base and an inert organic solvent. As illustrated in Scheme 4, chirality is introduced via Evans technology by employing an (S)-oxazolidinone derivative. Examples for suitable bases employed in the condensation include, but are not limited to, n-butyl lithium, lithium diisopropylamide (LDA), lithium hexamethyldisilazide, sodium hexamethyldisilazide and potassium hexamethyldisilazide. Commonly used solvents are for example tetrahydrofuran, dichloromethane, toluene or mixtures thereof. Step (b) is preferably performed at a temperature ranging from about -78°C to room temperature. Most preferably, the compound of formula (III) is reacted with allyl iodide or allyl bromide in tetrahydrofuran using lithium hexamethyl disilazide at a temperature of -70°C.

Steps (c1), (c2) and (c3):

Hydrolysis of esters or amides is known per se. Generally, such hydrolysis resulting in carboxylic acids or carboxylates is carried out with a metal hydroxide or alkoxide in an aqueous solvent mixture such as water/ethanol or water/DMF at temperatures between -30°C and the boiling point of the reaction mixture, or with a metal halogenide in the presence of a trimethylsilyl halogenide in a protic solvent such as acetonitrile or DMF at temperatures between 0°C and the boiling point of the reaction mixture.

By removal of the oxazolidinone group, the compound of formula (IV) is converted to a compound of formula (V) and the compound of formula (X) is converted to a compound of formula (VIII). Removal of the oxazolidinone group can generally be carried out according to methods well known in the art, e.g. using lithium peroxide in an organic solvent such as THF. Most preferably, steps (c1) and (c3) are carried out in a 3:1 (v/v) mixture of tetrahydrofuran and water at a temperature of 0°C using a mixture of LiOH and 30% $H_2O_2$.

Step (d):

The esterification of acids is known per se. For example, the esterification of acid (V) to carboxylic ester (VI) is carried out with an acid catalyst in an alcohol solvent at temperatures between -30°C and the boiling point of the reaction mixture.

Steps (e2), (e3), (e4) and (e5) :

Preferably, these reaction steps are carried out in the presence of a metathesis catalyst like a Grubbs catalyst such as $Cl_2(P(cymene)_3)_2Ru$=CHPh or Hoveyda-Grubbs 2$^{nd}$ generation catalyst, i.e. (1,3-Bis-(2,4,6-trimethylphenyl)-2-imidazolidinylidene)dichloro(o-isopropoxyphenylmethylene)ruthenium. The molar ratio of metathesis catalyst to compounds (IV), (V), (VI), (VII) or (XII) preferably ranges from 0.001:1 to 0.2:1, more preferably from 0.01:1 to 0.1:1 and most preferably from about 0.02:1 to about 0.05:1. Solvents suitable for the reactions of steps (e2) to (e5) include chlorinated hydrocarbons such as methylene chloride and the like, or aromatic hydrocarbons such as toluene and the like, or ionic liquids. Preferably, in steps (e2) to (e5) the reaction mixture is heated to a temperature ranging from room temperature to the boiling temperature of the reaction mixture. Most preferably, in steps (e2) and (e5) the compound of formula (VI) is converted into the compound of formula (IX) in the presence of $Cl_2(P(cymene)_3)_2Ru$=CHPh or Hoveyda-Grubbs 2$^{nd}$ generation catalyst using dichloromethane at reflux temperature.

Steps (g1) and (g2) :

The compounds of formula (V) and of formula (VIII) are preferably converted into an acid anhydride in the presence of a dehydrating agent. Suitable dehydrating agents include acetic anhydride, trifluoroacetic anhydride, dicyclohexylcarbodiimide, methoxyacetylene, $P_2O_5$ and mixtures thereof. Preferably, dicyclohexylcarbodiimide is used as dehydrating agent. The molar ratio of dehydrating agent to the compound of formula (V) or (VIII) preferably ranges from 0.1:1 to 1:1 and most preferably is about 0.4:1 to 0.6:1. The temperature for the reaction of steps (g1) and (g2) is preferably ranging from room temperature to the boiling temperature of the reaction mixture. Preferably, dichloromethane is used as solvent in steps (g1) and (g2).

Step (h) :

The hydrolysis of anhydrides are known per se. For example, the hydrolysis of anhydrides of formula (XIII) and (XIIIa) to a dicarboxylic acid (VIII) is carried out with a metal hydroxide or alkoxide in an aqueous solvent mixture such as water/ethanol or water/DMF at temperatures between -30°C and the boiling point of the reaction mixture, or with a metal halogenide preferably in the presence of a trimethylsilyl halogenide in a protic solvent such as acetonitrile or DMF at temperatures between 0°C and the boiling point of the reaction mixture.

In particular, in step (h) the mixture comprising the compounds of formulae (XIII) and (XIIIa) is converted to the compound of formula (VIII) in the presence of a metal hydroxide such as an alkaline metal hydroxide like NaOH.

Step (i3):

The compound of formula (XIII) is coupled with an organometallic reagent (XXIII) to provide compound of formula (XVI). In step (i3), also a dimer of the compound of formula (XIII) can be used as starting material. A preferred dimer is represented by formula (XIIIa) in Scheme 3. In one embodiment, a mixture of compounds of formulae (XIII) and (XIIIa) as obtained in step (e2) is used as starting material of step (i3). The following description of preferred embodiments shall apply to step (i3). Preferably, the organometallic reagent, i.e. the compound of formula (XXIII), is selected from the group consisting of 4-methoxy-3-(3-methoxypropyl)phenyl magnesium bromide, 4-methoxy-3-(3-methoxypropyl)phenyl magnesium chloride, 4-methoxy-3-(3-methoxypropyl)phenyl magnesium iodide, 4-methoxy-3-(3-methoxypropyl)phenyl lithium, 4-methoxy-3-(3-methoxypropyl)phenyl zinc bromide and 4-methoxy-3-(3-methoxypropyl)phenyl zinc chloride. The organometallic reagent can be used in excess of 1 to 5 moles, preferably 1 to 2 moles, more preferably 1.1 to 1.4 moles per mole of the compound of formulae (XIII). Preferably, step (i3) is performed in an inert solvent which is more preferably selected from the group consisting of tetrahydrofuran, 2-methyltetrahydrofuran, diglyme, dioxane, diethyl ether, diisopropyl ether, tert-butyl methyl ether, cyclopentyl methyl ether, toluene and mixtures thereof. Most preferably, tetrahydrofuran and/or toluene are used as inert solvent. The reaction temperature is preferably below the boiling temperature of the solvent used, in particular between -78°C to the boiling temperature of the solvent, more preferably between -78°C to 35°C. It is further preferred that the reaction is carried out for about 0.5 to 4 hours, preferably 1 hour. After completion of the reaction, the reaction mixture is acidified and extracted with a suitable solvent.

It is particularly preferred that as starting material of step (i3) the compound of formula (XIII) is used in the form of the (E) stereoisomer, i.e. (3S, 8S, E)-3,8-diisopropyl-3,4,7,8-tetrahydrooxonine-2,9-dione having the following formula (XIIIb)

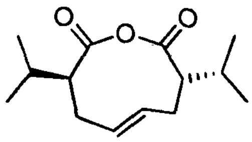

(XIIIb).

According to one embodiment of step (i3), a process is excluded which comprises the following steps: adding dropwise a 3 M solution of 4-methoxy-3-(3-methoxypropyl)phenyl magnesium bromide in $Et_2O$ (140 mL) to a solution of (3S,8S,E)-3,8-diisopropyl-3,4,7,8-tetrahydrooxonine-2,9-dione (100 g, 0.419 mol) in THF (1.5 L) at 0°C; stirring the reaction mixture at room temperature for 24 h; slowly adding 1M HCl (1.5 L) and extracting with EtOAc (3 x 500 mL); evaporating the solvent to obtain crude (2S,7S,E)-2-isopropyl-7-(4-methoxy-3-(3-methoxypropoxy)benzoyl)-8-methylnon-4-enoic acid (95.4 g).

Step (j1):

Preferably, in step (j1) the compound of formula (XVI) is lactonized in the presence of a halogenation agent selected from the group consisting of $Br_2$, $I_2$, N-halogenated carboxamides, N-halogenated dicarboxamides, N-halogenated phthalimides, N-halogenated succinimides, N-halogenated sulfonamides, N-halogenated imides, hypochlorites and mixtures thereof. In particular, suitable halogenation agents include, but are not limited to, elemental bromine and iodine, in particular N-chloro, N-bromo and N-iodocarboxamides and dicarboximides. Preferred are N-chloro, N-bromo and N-iodophthalimide and especially N-chloro, N-bromo and N-iodosuccinimide, as well as tertiary butyl hypochlorite and N-halogenated sulfonamides and imides, for example chloroamine T.

It is preferred to carry out step (j1) in organic solvents, more preferably water-miscible solvents, for example tetrahydrofuran or dioxane. The reaction temperature can range from about -70°C to ambient temperature.

Furthermore, step (j1) is advantageously carried out in the presence of at least one inorganic or organic

acid and at least one equimolar amount of water, based on the compound of formula (XVI), respectively. Examples of suitable acids include formic acid, acetic acid, methanesulfonic acid, trifluoroacetic acid, trifluoromethanesulfonic acid, toluenesulfonic acid, $H_2SO_4$, $H_3PO_4$, HCl and HBr.

Most preferably, step (j1) is carried out using N-bromosuccinimide in the presence of glacial acetic acid and a mixture of tetrahydrofuran and water as a solvent.

According to one embodiment of step (j1), a process is excluded which comprises the following steps: adding N-bromosuccinimide (29.3 g, 165 mmol, 1.05 equiv) in small portions over 10 min to a solution of (2S,7S,E)-2-isopropyl-7-(4-methoxy-3-(3-methoxypropoxy)benzoyl)-8-methylnon-4-enoic acid (68.3 g, 157 mmol, 1 equiv) and glacial acetic

acid (44.9 mL, 784 mmol, 5.0 equiv) in a 4:1 mixture of THF and $H_2O$ (1,65 L) at 0 °C; stirring the resulting yellow solution for 15 min at 0 °C; then warming it to 23 °C and subsequently refluxing it for 45 min; after cooling to 23 °C, partitioning the reaction mixture between half-saturated $NaHCO_3$ (2 L) and a 1:1 mixture of EtOAc and hexanes (2 x 2 L); drying the combined organic layers over $Na_2SO_4$ and concentrating; and purifying the residue by flash chromatography (gradient elution, 5-10% EtOAc in hexanes) to give (3S,5S)-5-((1R,3S)-1-bromo-3-(4-methoxy-3-(3-methoxypropoxy)benzoyl)-4-methylpentyl)-3-isopropyldihydrofuran-2(3H)-one (52.5 g).

Step (k1):

Preferably, step (k1) is carried out in a mixture of an organic solvent and water in the presence of an azidation agent. In particular, the reactions are carried out in water-miscible organic solvents mixed with water, typically for example alcohols or ethers such as methanol, ethanol, ethylene glycol, diethylene glycol, diethylene glycol monomethyl or ethyl ether, diethyl ether, tetrahydrofuran or dioxane. In case $R_5$ of compound (XVIII) is $-N_3$, the conversion of step (k1) is an azidation. Examples of azidation agents useful in step (k1) include metal azides, especially alkaline earth metal azides and alkali metal azides, as well as silyl azides. Especially preferred azidation agents are lithium azide, sodium azide and potassium azide. Moreover, it is preferred that step (k1) is carried out in the presence of a phase transfer catalyst. The reaction temperature of step (k1) is preferably from 30 to 70°C like from 45 to 55°C.In case $R_5$ of compound (XVIII) is $-NR_7R_8$, it is preferred to firstly perform an azidation as described above and then to convert the azido group into the amino group $-NR_7R_8$.

Most preferably, step (k1) is carried out in dimethylformamide in the presence of $NaN_3$.

According to one embodiment of step (k1), a process is excluded which comprises the following steps: heating a suspension of sodium azide (16.25 g, 250 mmol, 2.5 equiv) and (3S,5S)-5-((1R,3S)-1-bromo-3-(4-methoxy-3-(3-methoxypropoxy)benzoyl)-4-methylpentyl)-3-isopropyldihydrofuran-2(3H)-one (51.35 g, 100 mmol, 1 equiv) in DMF (400 mL) at 50 °C for 67 h; cooling the reaction mixture to 23. °C; partitioning between saturated NaCl (2 L) and a 1:1 mixture of EtOAc, hexanes (2 x 2 L); drying the combined organic layers over $Na_2SO_4$ and concentrating; purifying the residue by flash column chromatography (gradient elution, 5-20% EtOAc in hexanes) to give (3S,5S)-5-((1S,3S)-1-azido-3-(4-methoxy-3-(3-methoxypropoxy)benzoyl)-4-methylpentyl)-3-isopropyldihydrofuran-2(3H)-one (32.4 g).

Step (l) :

The reaction of a compound of formula (XVIII) with a compound of formula (XIV) by opening of the lactone ring is usually carried out in the presence of alcohols or amines which are capable of forming activated carboxylic acid esters or carboxamides. Such compounds are well-known. Examples therefor include 2-hydroxypyridine, N-hydroxycarboxamides and imides, and carboximides such as N-hydroxysuccinimide. Organic solvents are used as solvent, with tertiary amines being of advantage, for example trimethyl or triethyl amines. The reaction temperature may range for example from about 40°C to 150°C.

Most preferably, step (1) is carried out in the presence of 2-hydroxypyridine using triethyl amine at a temperature of about 80°C.

According to one embodiment of step (1), a process is excluded which comprises the following steps: refluxing a mixture of (3S,5S)-5-((1S,3S)-1-azido-3-(4-methoxy-3-(3-methoxypropoxy)benzoyl)-4-methylpentyl)-3-isopropyldihydrofuran-2(3H)-one (29.0 g, 49 mmol), 3-amino-2,2-dimethylpropionamide (11.38 g, 98 mmol) and 2-hydroxypyridine (1.1 g) in triethylamine (30 mL) for 24 hrs; pouring the cooled reaction mixture into saturated $NaHCO_3$ (500 mL) and extracting with EtOAc (3 x 250 mL); washing the combined organic layers with saturated $NaHCO_3$ (500 mL) and saturated NaCl (500 mL); drying over $Na_2SO_4$; evaporating the solvent to obtain (2S,4S,5S,7S)-N-(3-amino-2,2-dimethyl-3-oxopropyl)-5-azido-4-hydroxy-2-isopropyl-7-(4-methoxy-3-(3-methoxypropoxy)benzoyl)-8-methylnonanamide as an oil (32.3 g).

Step (m):

In particular, in step (m) a compound of formula (XIX), wherein $R_5$ is $-N_3$, is converted to aliskiren. In such an embodiment, the azido group $R_5$ is reduced to $-NH_2$ and, at the same time or subsequently, the benzoyl group is reduced to a benzyl group. In case $R_5$ in the compound of formula (XIX) is an amino group or protected amino group, such a group is converted into $-NH_2$ prior to or after the reduction of the benzoyl group. Likewise, in case $R_6$ in the compound of formula (XIX) is a hydroxyl protecting group, such protecting group is removed prior to or after the reduction of the benzoyl group. Preferably, the conversion of the compound of formula (XIX) to aliskiren is performed using metal hydrides such as LiH, NaH, $LiAlH_4$, $NaBH_4$ or more preferably using hydrogen in the presence of a catalyst. The catalyst is preferably a homogeneous or heterogeneous catalysts and can more preferably be selected from the group consisting of Wilkinson catalyst, Raney nickel, precious metal catalysts such as platinum and palladium, optionally on substrates

such as carbon. The hydrogenation can also be carried out catalytically under phase transfer conditions, for example with ammonium formate as hydrogen donor. In step (m), it is advantageous to use an organic solvent. In particular, the organic solvent is an aliphatic alcohol such as methanol, ethanol, isopropanol or tert.-butyl alcohol, and preferably is ethanol. The reaction temperature may range for example from approximately 0° C to 200° C and preferably ranges from 10 to 50°C, more preferably from 20 to 30°C. Hydrogenation may be carried out at normal pressure or increased pressure up to 100 bar. Preferably, the pressure ranges from 1.5 to 10 bar like 3 to 5 bar. The reaction can also be catalyzed by addition of an acid catalyst, preferably conc. HCl or perchloric acid.

According to one embodiment of step (m), a process is excluded which comprises the following steps: dissolving (2S,4S,5S,7S)-N-(3-amino-2,2-dimethyl-3-oxopropyl)-5-azido-4-hydroxy-2-isopropyl-7-(4-methoxy-3-(3-methoxypropoxy)benzoyl)-8-methylnonanamide (30.1 g, 50.8 mmol) in ethanol (600 mL) and conc. HCl (2 mL) and hydrogenating in the presence of 5% Pd/C (3.6 g) at ambient temperature and 4.0 bar; filtering the reaction mixture; washing the catalyst with ethanol (100 mL); evaporating the solvent and dissolving the residue in water (500 mL); adjusting pH to 12 by addition of 1M NaOH; extracting the solution with tert.-butyl methyl ether (TBME) (4 x 300 mL); drying the combined organic phases over $Na_2SO_4$ and evaporating the solvent; adding ethanol (100 mL) and fumaric acid (2.95 g) and filtering; concentrating the filtrate and dissolving it in acetonitrile (850 mL) at 35°C; stirring the resulting solution at room temperature for 24 hrs, then 2 hrs at 0°C and filtering off; washing the residue with acetonitrile (3 x 80 mL) and drying in a vacuum at 35°C to obtain aliskiren hemifumarate as white crystals.

As mentioned above, the present invention also includes any variant of the above process steps, in which the reaction components are used in the form of their salts or solvates or in a protected form.

As also mentioned above, the present invention also includes any process comprising step (i3) and other step described above.

It is also disclosed any process, in which an intermediate product obtainable by any reaction step mentioned above is used as the starting material, and the remaining steps described above or other steps are carried out to arrive at aliskiren of formula (I) or a pharmaceutical acceptable salt thereof.

Thus, it is also disclosed a process for preparing aliskiren or a pharmaceutically acceptable salt thereof comprising at least one reaction step selected from the group consisting of step (e2), i.e. converting compound (XII) into compounds (XIII) and (XIIIa), step (g1), i.e. converting compound (VIII) into compound (XIII), step (h), i.e. converting compounds (XIII) and (XIIIa) into compound (VIII), step (i3), i.e. converting compound (XIII) into compound (XVI), step (l), i.e. converting compound (XVIII) into compound (XIX), step (m), i.e. converting compound (XIX) into compound (I), and combinations of these reaction steps.

In a particularly preferred embodiment, the process according to the invention for preparing aliskiren comprises a combination of step (i3) and step (m), i.e. it is particularly preferred to firstly carry out step (i3) resulting in a compound of formula (XVI), converting the compound of formula (XVI) into a compound of formula (XIX) and carrying out step (m) to convert the compound of formula (XIX) to aliskiren or a salt thereof.

In this embodiment, the conversion of the compound of formula (XVI) to the compound of formula (XIX) preferably comprises steps (j1), (k1) and (1) described above. Moreover, it is preferred that in this embodiment the compound of formula (XIII) is prepared by using a compound of formula (V) as starting material and carrying out either reaction steps (g2) and (e2) described above or reaction steps (g2), (e2), (h) and (g1) described above.

Furthermore, the invention is directed to a compound of formula (XIII) as described above, a compound of formula (XIIIa) as described above, and a compound of formula (XIIIb) as described above. Moreover, the use of the compounds (XIII), (XIIIa) and (XIIIb) for the manufacture of aliskiren or a pharmaceutically acceptable salt thereof are further objects of the invention.

In addition, the invention is directed to a process for preparing aliskiren or a pharmaceutically acceptable salt thereof using at least one compound of formulae (XIII), (XIIIa) and (XIIIb). Preferably, this process comprises

providing at least one compound of formulae (XIII), (XIIIa) and (XIIIb), and

converting said compound(s) into aliskiren or a pharmaceutically acceptable salt thereof.

Further, the invention is also directed to a process for preparing a pharmaceutical composition containing aliskiren or a pharmaceutically acceptable salt thereof which process comprises preparing aliskiren or a pharmaceutically acceptable salt thereof in accordance with a process according to the invention described above and converting the aliskiren or salt thereof into a pharmaceutical composition. Formulations of aliskiren are known from e.g. EP-A-0 678 503, EP-A-1 517 682 and WO-A-2005/089729. Preferably, the conversion of aliskiren or pharmaceutically acceptable salt thereof into a pharmaceutical composition is effected by a hot-melt and solvent-free granulation process. In this connection, the term "solvent" denotes a compound which is liquid at room temperature and standard pressure. Hot-melt granulation of aliskiren is for example described in co-pending application EP 07 006 055.3.

Aliskiren or pharmaceutically acceptable salts thereof can also be used in pharmaceutical compositions that exhibit synergistic effects of aliskiren in combination with ACE inhibittors, ARBs, antidiabetics or diuretics which have been previ ously described in e.g. EP-A-1 341 533, EP-A-1 602 370, EP-A-1 507 558, US-A-2003/0114389, WO-A-03/099279, WO-A-2005/070406, and WO-A-2005/077418.

The invention is further illustrated with reference to the following examples and figures.

Figure 1 is an X-ray powder diffractogram of aliskiren hemifumarate obtained according to Example 1.

Figure 2 is an FT-IR spectrum of aliskiren hemifumarate obtained according to Example 1.

Figure 3 is a DSC thermogram of alsiskiren hemifumarate obtained according to Example 1.

Figures 4 and 5 are images of aliskiren hemifumarate obtained according to Example 1 which were recorded using optical microscopy.

Figure 6 is an X-ray powder diffractogram of aliskiren hemifumarate obtained according to Example 2.

Figure 7 is a DSC thermogram of aliskiren hemifumarate obtained according to Example 2.

## Examples

Example 1: Preparation of aliskiren hemifumarate

9 g of aliskiren were dissolved in 26 ml of ethanol and mixed with 0.95 g of fumaric acid. The mixture was filtered off and the solvent was evaporated. The residue was dissolved in 220 ml of acetonitrile and stirred at ambient temperature. The resulting solution was seeded with 50 mg of aliskiren hemifumarate and agitated at ambient temperature for 17 hours. The suspension was cooled to -10°C and filtered off by suction after 1 hour. The residue was washed with 3 x 25 ml of acetonitrile and then dried in a vacuum oven at 30°C for 1 hour. The aliskiren hemifumarate was obtained as white crystals (7.6 g).

$^{1}$H-NMR (DMSO-d6) $\delta$/ppm: 7.62 (t, 1H), 7.20 (s, 1H), 6.83-6.79 (m, 3H), 6.69 (dd, 1H), 6.35 (s, 1H), 3.97 (t, 2H), 3.71 (s, 3H), 3.46 (t, 2H), 3.28 (m, 1H), 3.23 (s, 3H), 3.18-3.08 (m, 2H), 2.57 (m, 1H), 2.46-2.25 (m, 3H), 1.92 (m, 2H), 1.79 (m, 1H), 1.69-1.52 (m, 3H), 1.41-1.21 (m, 3H), 1.03 (s, 6H), 0.85-0.76 (m, 12H).

X-ray powder diffraction:

A sample of the obtained aliskiren hemifumarate was measured by X-ray powder diffractometry. The XRD pattern was obtained on a Philips PW3040/60 X'Pert powder diffractometer using X'celerator detector at CuK$\alpha$ radiation, 1.54178 Å, 3°<2$\theta$<30°. The obtained X-ray powder diffractogram is shown in Figure 1 and is characterized by the following peaks:

| No. | Pos. [°2Th.] | d-spacing [A] | Rel. Int. [%] |
|---|---|---|---|
| 1 | 7.3 | 12.07 | 47 |
| 2 | 10.0 | 8.89 | 67 |
| 3 | 11.0 | 8.01 | 55 |
| 4 | 14.9 | 5.93 | 41 |
| 5 | 17.8 | 4.97 | 58 |
| 6 | 19.1 | 4.65 | 95 |
| 7 | 20.0 | 4.44 | 100 |
| 8 | 21.6 | 4.11 | 66 |

FT-IR spectroscopy:

FT-IR spectra of KBr discs prepared from the obtained aliskiren hemifumarate were recorded over a wave number range of 4000-400 $cm^{-1}$ on a Perkin Elmer FT-IR spectrometer Spectrum GX at a resolution of 4 $cm^{-1}$. The obtained FT-IR spectrum is shown in Figure 2.

Thermal analysis:

DSC thermograms were recorded on a DSC 822e Mettler Toledo scanning calorimeter. Samples of approx. 3 mg were scanned between 20°C and 200°C at a heating rate of 10°C/min under nitrogen atmosphere. The obtained DSC thermogram is shown in Figure 3.

Microscopy:

Images of particles of the obtained aliskiren hemifumarate were taken on a Microscope Olympus BX 50 equipped with Olympus camera DP70. The sample was prepared as suspension of aliskiren hemifumarate in paraffinic oil. The obtained images are shown in Figures 4 and 5.

Example 2: Preparation of amorphous aliskiren hemifumarate

Aliskiren hemifumarate obtained according to Example 1 was milled using a ball mill at 20 Hz for 30 min.

An X-ray powder diffractogram of the obtained amorphous aliskiren hemifumarate is shown in Figure 6. Moreover, the corresponding DSC thermogram is shown in Figure 7.

Example 3: Preparation of (S)-4-benzyl-3-(3-methylbutanoyl)oxazolidin-2-one

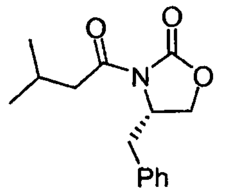

To a cold suspension (-15 °C) of sodium hydride (7.12 g, 178 mmol; ~60% oil suspension) in anhydrous THF (600 mL) (S)-4-benzyloxazolidin-2-one (30.0 g, 169.3 mmol) was added portionwise. The reaction mixture was allowed to reach room temperature during 2 h and stirring was continued for 20 h, after the mixture was recooled to -15 °C once more. 3-Methylbutanoyl chloride (23.48 g, 24.00 mL, 194.7 mmol) was added dropwise and the mixture was allowed to reach room temperature during 2 h, and sitirring was continued for additional 3 h. The reaction was quenched by addition of saturated aqueous solution of $NaHCO_3$ (300 mL) and the mixture was stirred at room temperature for 30 min. The product was extracted with $CH_2Cl_2$ (3 × 400 mL). The combined organic layers were washed with saturated aqueous $Na_2CO_3$ solution (2 × 300 mL) and brine (300 mL), and dried over anhydrous $Na_2SO_4$. After removal of the solvents under reduced pressure the oily residue was recrystallized from $Et_2O$/petrolether 1:10 (408 mL) to yield, in two crops, 37.19 g (84%) of (*S*)-4-benzyl-3-(3-methylbutanoyl)oxazolidin-2-one as colourless crystals. $^1H$ NMR (300 MHz, $CDCl_3$) δ: 1.03 (d, 3H), 1.04 (d, 3H), 2.24 (m, 1H), 2.75 (dd, 1H) 2.73-2.95 (m, 1H), 3.32 (dd, 1H), 4.14-4.23 (m, 2H), 4.66-4.74 (sym m, 1H), 7.22-7.38 (m, 5H).

Example 4: Preparation of (S)-3-((S)-2-isopropylpent-4-enoyl)-4-benzyloxazolidin-2-one

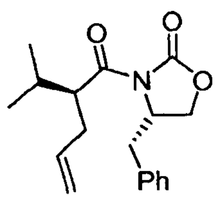

(a) Alkylation with allyl bromide: To a solution of lithium diisopropylamide (23 mL, 46 mmol; 2 M in THF) in anhydrous THF (120 mL), stirred at -78 °C under argon, a solution of (*S*)-4-benzyl-3-(3-methylbutanoyl)oxazolidin-2-one (10.0 g, 38.27 mmol) in anhydrous THF (10 mL) was added and the mixture was stirred for 1.5 h. Then allyl bromide (16.22 g, 134.07 mmol) and DMPU (16 mL) were successively added dropwise. The resulting mixture was continued to stir at -78 °C for 2 h and then allowed to reach -45 °C at which it was stirred for 4 h. The mixture was allowed to warm to 10 °C during 12 h. The reaction was quenched by the addition of saturated aqueous $NH_4Cl$ solution (100 mL) and the product was extracted with ethyl acetate (3 × 250 mL). The combined organic layers were successively washed with ice-cold HCl (1 M, 100 mL), saturated aqueous $NaHCO_3$ solution (2 × 100 mL), and brine (2 × 100

mL), and then dried over anhydrous $Na_2SO_4$. After removal of the solvents under reduced pressure 14.46 g of crude product were obtained which were further purified by radial chromatography on silica gel (petrolether/ethyl acetate = 10:1) to afford 8.994 g (78 %) of alkylation product as a yellowish oil.

(b) Alkylation with allyl iodide: To a solution of lithium diisopropylamide (23 mL, 46 mmol; 2 M in THF) in anhydrous THF (120 mL), stirred at -78 °C under argon, a solution of (*S*)-4-benzyl-3-(3-methylbutanoyl)oxazolidin-2-one (10.0 g, 38.27 mmol) in anhydrous THF (10 mL) was added and the mixture was stirred for 1.5 h. To the resulting mixture allyl iodide (10.30 g, 61.32 mmol) was added dropwise and stirring was continued for another 30 min. The resulting mixture was warmed to -45 °C at which it was stirred for 15 h. The reaction was quenched by the addition of 1 M aqueous HCl solution (100 mL) and the product was extracted with ethyl acetate (3 × 250 mL). The combined organic layers were successively washed with ice-cold 1 M aqueous HCl solution (100 mL), saturated aqueous $NaHCO_3$ solution (2 × 100 mL), and brine (2 × 100 mL), and dried over anhydrous $Na_2SO_4$. After removal of the solvents under reduced pressure 11.952 g of crude product were obtained which were further purified by radial chromatography on silica gel (petrolether/ethyl acetate = 10:1) to afford 8.302 g (72%) of alkylation product as a colourless oil. $^1H$ NMR (300 MHz, $CDCl_3$) δ : 0.98 (d, 6H), 1.98 (m, 1H), 2.35-2.54 (m, 2H), 2.64 (dd, 1H), 3.31 (dd, 1H), 3.83-3.90 (sym m, 1H), 4.12-4.17 (m), 4.65-4.73 (m, 1H), 5.02 (d, 1H), 5.09 (ddt 1H), 5.76-5.90 (m, 1H), 7.22-7.36 (m, 5H).

Example 5: Preparation of (*S*)-2-isopropylpent-4-enoic acid

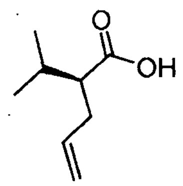

A solution of (S)-3-((S)-2-isopropylpent-4-enoyl)-4-benzyloxazolidin-2-one (1.753 g, 5.82 mmol) in THF (30 mL) and $H_2O$ (10 mL), stirred at 0 °C, was treated with ~30% aqueous $H_2O_2$ solution (4 mL) followed by LiOH (0.489 g, 11.65 mmol) in $H_2O$ (5 mL). The resulting mixture was warmed to room temperature at which it was stirred for 5 h. The mixture was recooled to 0 °C and 2 M aqueous $Na_2SO_3$ solution (10 mL) was added. Then, THF was evaporated under reduced pressure. The aqueous residue was basified with 2 M aqueous NaOH solution to pH 12-13 and extracted with $CH_2Cl_2$ (5 × 20 mL). The aqueous layer was cooled to 0 °C and acidified with 10% aqueous HCl solution to pH ~1. The product was extracted with ethyl acetate (5 × 20 mL), and the combined organic layers were dried over anhydrous $Na_2SO_4$. After removal of the solvent 0.732 g (88%) of (*S*)-2-isopropylpent-4-enoic acid were obtained as a colourless oil. $[\alpha]_{Hg}^{25} = +13.2$ (c = 1.14, $CH_2Cl_2$). .$^1H$ NMR (300 MHz, $CDCl_3$) δ: 0.98 (d, 3H), 1.00 (d, 3H), 1.93 (m, 1H), 2.20-2.40 (m, 3H) 5.02 (d, 1H), 5.08 (ddt 1H), 5.71-5.85 (m, 1H), 9.99 (m, 5H).

Example 6: Preparation of (2*S*)-2-isopropylpent-4-enoic anhydride

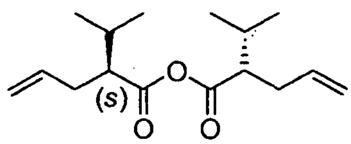

To a solution of (*S*)-2-isopropylpent-4-enoic acid (1.0 g, 7.04 mmol) in dry dicloromethane (17 mL) a solution of N,N'-dicyclohexylcarbodiimide (0.747 g, 3.62 mmol) in dry dichloromethane (8 mL) was added over a period of 6 hours. The reaction mixture was continued to stir for an additional hour at room temperature. After the reaction was complete, the reaction mixture was filtered and the solvent was evaporated at 40 °C under reduced pressure. The residue

was suspended in petroleum ether (30 mL) and cooled to -20 °C for 8 hours. The precipitated material was filtered off and washed with petroleum ether (10 mL). The solvent was removed by evaporation under the reduced pressure at 40 °C and 1.06 g of colourless oily residue were distilled under vacuum. $[\alpha]^{25}_{Hg} = +12.6.$ (c = 5.0, $CH_2Cl_2$). $^1H$ NMR (300 MHz, $CDCl_3$) δ: 1.00 (d, 3H), 1.01 (d, 3H), 1.97 (m, 1H) 2.25-2.41 (m,3H), 5.03-5.14 (m, 2H), 5.71-5.85 (m, 1H).

Example 7: Preparation of methyl (2*S*)-2-isopropylpent-4-enoate

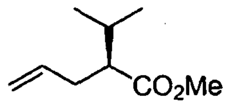

To a solution of (*S*)-2-isopropylpent-4-enoic acid (5.265 g, 37.1 mmol) in methanol (120 mL) concentrated $H_2SO_4$ (840 mg) was added at room temperature. The reaction mixture was refluxed for 24 hours. After the reaction was complete the reaction mixture was evaporated at 25 °C under reduced pressure and the residue was dissolved in dichloromethane (100 mL), and washed with saturated aqueous solution of $NaHCO_3$ (3×100 mL). Aqueous phase was extracted with dichloromethane (1×50 mL) and combined organic layers were dried over anhydrous $Na_2SO_4$ and evaporated at 25 °C, yielding pure compound as a colourless oil, 4.0 g. $^1H$ NMR (300 MHz, $CDCl_3$) δ: 0.92 (d, 3H), 0.95 (d, 3H), 1.88 (m, 1H), 2.19-2.34 (m, 3H), 3.65 (s, 3H,), 4.98 (m, 1H), 5.05 (m, 1H), 5.74 (m, 1H).

Example 8: Preparation of dimethyl (2*S*,7*S*)-2,7-diisopropyloct-4-enedioate

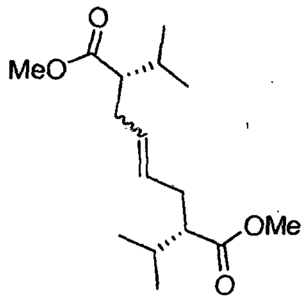

To a solution of methyl (*S*)-2-isopropylpent-4-enoate (2.5 g, 16.0 mmol) in dichloromethane (160 mL) Hoveyda-Grubbs catalyst 2nd generation catalyst (480 mg, 5 mol%) was added. The reaction mixture was refluxed for 24 hours and then evaporated at 25 °C. The residue was purified by column chromatography ($SiO_2$, petroleum ether/ethyl acetate = 25:1) yielding 1.543 g of pure product as a colourless oil. $^1H$ NMR (300 MHz, $CDCl_3$) δ: 0.84 (d, 6H), 0.88 (d, 6H), 1.79 (m, 2H), 2.07-2.21 (m, 6H), 3.60 (s, 6H), 5.33 (m, 2H). HRMS calculated for $C_{16}H_{28}O_4$: 284.1988; found: 284.1996.

Example 9: Preparation of (3S,8S)-3,8-diisopropyl-3,4,7,8-tetrahydrooxonine-2,9-dione

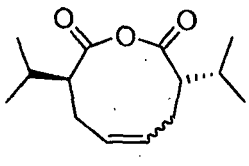

+

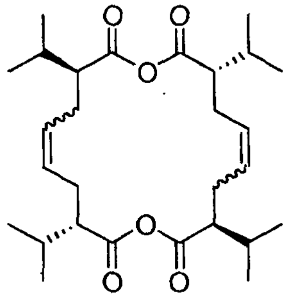

A

B

Hoveyda-Grubbs 2nd generation catalyst (89 mg, 2.5 mol%) was added to a solution of (2S)-2-isopropylpent-4-enoic anhydride (1.0 g, 3.76 mmol) in dichloromethane (40 mL). The reaction mixture was refluxed for 5 hours and then evaporated at 25 °C. The residue was purified by column chromatography ($SiO_2$, petroleum ether/ethyl acetate = 25/1) yielding 237 mg of a mixture of title compounds A and B in a ratio of 1:9 = A:B. $^1H$ NMR (300 MHz, $CDCl_3$) $\delta$ 0.98 (t, J = 7.0 Hz, 12H), 1.92 (sim m, J = 7.0 Hz, 2H), 2.16-2.35 (m, 6H), 5.46 (m, 2H). Compound A: MS (CI) m/z (%): 239 (MH+, 20), 121 (75), 155 (100). HRMS Calculated for C14H2303: 239.1647; found: 239.1650. Compound B: MS (ESI) m/z (%): 499 (M + Na+); HRMS Calculated for C28H4406Na: 499.3036; found: 499.3012.

Example 10: Preparation of (2S,7S)-2,7-Diisopropyloct-4-enedioic acid

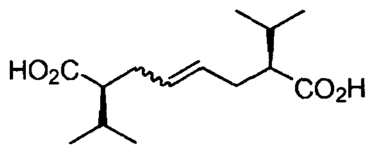

Hoveyda-Grubbs 2nd generation catalyst (89 mg, 2.5 mol%) was added to a solution of (2S)-2-isopropylpent-4-enoic anhydride (1.0 g, 3.76 mmol) in dichloromethane (40 mL). The reaction mixture was refluxed for 12 hours and then evaporated at 25 °C. The residue was dissolved in tetrahydrofuran (15 mL) and NaOH (2M, 6 mL) was added. The reaction mixture was stirred at room temperature for 24 hours. After reaction was completed the reaction mixture was evaporated under reduced pressure and the obtained residue was dissolved in dichloromethane (20 mL) and washed with NaOH solution (2M, 30 mL). The water layer was acidified with HCl (1/1, v/v) to pH = 1 and the product was extracted with ethyl acetate (3$\times$30 mL). The combined organic phases were dried over anhydrous $Na_2SO_4$, filtered and the filtrate was evaporated yielding 914 mg (95%) of product. $^1H$ NMR (300 MHz, CDCl3), $\delta$ 0.96 (d, 7.0 Hz, 12H), 1.90 (sept, 6.5 Hz, 2H), 2.18-2.28 (m, 6H), 5.41-5.47 (m, 2H), 9.63 (br s, 2H). MS (ESI) m/z (%): 279 (M + Na+); HRMS Calculated for C14H2404Na: 279.1562; found: 279.1572.

Example 11: Preparation of (2S,7S)-2,7-diisopropyloct-4-enedioyl dichloride

To a solution of (2S,7S)-2,7-Diisopropyloct-4-enedioic acid (10.3 g) in dichloromethane (100 ml) was added 2M solution of oxalyl chloride in dichloromethane (61 ml) and the mixture was stirred at room temperature for 16.5 h. Concentration in vacuo gave the acid chloride (11.7 g, quant.) as a viscous amber colored oil.

Example 12: Preparation of (4*S*)-4-benzyl-3-[(2*S*,7*S*)-7-{[(4*S*)-4-benzyl-2-oxo-1,3-oxazolidin-3-yl]carbonyl}-2-isopropyl-8-methylnon-4-enoyl]-1,3-oxazolidin-2-one

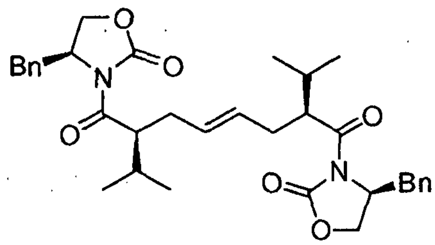

To a solution of (4*S*)-4-benzyl-3-[(2*S*)-2-isopropylpent-4-enoyl]-1,3-oxazolidin-2-one (600 mg, 2.00 mmol) in dichloromethane (40 mL) Hoveyda-Grubbs 2nd generation catalyst (64 mg, 5 mol%) was added. The reaction mixture was refluxed for 24 hours and then evaporated at 25 °C. The residue was purified by column chromatography ($SiO_2$, petroleum ether-ethyl acetate = 10/1) yielding 176 mg of pure product, colourless oil. $^1H$ NMR (300 MHz, CD3Cl) *major isomer*: 0.94 (t, 12H), 1.97 (m, 2H), 2.26-2.47 (m, 4H), 2.70 (dd, 2H), 3.33 (dd, 2H), 3.78 (m, 2H), 4.11 (m, 4H), 4.66 (m, 2H), 5.53 (m, 2H); 7.18-7.35 (m, 5H,); *minor isomer*: 0.94 (t, 12H), 1.97 (m, 2H), 2.26-2.47 (m, 4H), 2.61 (dd, 2H), 3.24 (dd, 2H), 3.78 (m, 2H), 4.11 (m, 4H), 4.66 (m, 2H), 5.46 (m, 2H), 7.18-7.35 (m, 5H). HRMS ($C_{34}H_{42}N_2O_6$); calculated: 575.3121; found: 575.3130.

<u>Example 13:</u> Preparation of (2S,7S,E)-2-isopropyl-7-(4-methoxy-3-(3-methoxypropoxy)benzoyl)-8-methylnon-4-enoic acid

To a solution of (3S,8S,E)-3,8-diisopropyl-3,4,7,8-tetrahydrooxonine-2,9-dione (100 g, 0.419 mol) in THF (1.5 L) at 0°C, a 3 M solution of 4-methoxy-3-(3-methoxypropyl)phenyl magnesium bromide in $Et_2O$ (140 mL) was added dropwise, and the reaction mixture was stirred at room temperature for 24 h. Then, 1M HCl was slowly added (1.5 L) and extracted with EtOAc (3 x 500 mL). The solvent was evaporated to obtain crude (2S,7S,E)-2-isopropyl-7-(4-methoxy-3-(3-methoxypropoxy)benzoyl)-8-methylnon-4-enoic acid (95.4 g).

<u>Example 14:</u> Preparation of (3S,5S)-5-((1R,3S)-1-bromo-3-(4-methoxy-3-(3-methoxypropoxy)benzoyl)-4-methylpentyl)-3-isopropyldihydrofuran-2(3H)-one

N-Bromosuccinimide (29.3 g, 165 mmol, 1.05 equiv) was added in small portions over 10 min to a solution of (2S,7S,E)-2-isopropyl-7-(4-methoxy-3-(3-methoxypropoxy)benzoyl)-8-methylnon-4-enoic acid (68.3 g, 157 mmol, 1 equiv) and glacial acetic acid (44.9 mL, 784 mmol, 5.0 equiv) in a 4:1 mixture of THF and $H_2O$ (1,65 L) at 0 °C. The resulting yellow solution was stirred for 15 min at 0 °C, then it was warmed to 23 °C, and subsequently refluxed for 45 min. After cooling to 23 °C, the reaction mixture was partitioned between half-saturated $NaHCO_3$ (2 L) and a 1:1 mixture of EtOAc and hexanes (2 x 2 L). The combined organic layers were dried over $Na_2SO_4$ and concentrated. Flash chromatographic purification of the residue (gradient elution, 5-10% EtOAc in hexanes) gave (3S,5S)-5-((1R,3S)-1-bromo-3-(4-methoxy-3-(3-methoxypropoxy)benzoyl)-4-methylpentyl)-3-isopropyldihydrofuran-2(3H)-one (52.5 g).

<u>Example 15:</u> Preparation of (3S,5S)-5-((1S,3S)-1-azido-3-(4-methoxy-3-(3-methoxypropoxy)benzoyl)-4-methylpentyl)-3-isopropyldihydrofuran-2(3H)-one

A suspension of sodium azide (16.25 g, 250 mmol, 2.5 equiv) and (3S,5S)-5-((1R,3S)-1-bromo-3-(4-methoxy-3-(3-methoxypropoxy)benzoyl)-4-methylpentyl)-3-isopropyldihydrofuran-2(3H)-one (51.35 g, 100 mmol, 1 equiv) in DMF (400 mL) was heated at 50 °C for 67 h. The reaction mixture was cooled to 23 °C and partitioned between saturated NaCl (2 L) and a 1:1 mixture of EtOAc, hexanes (2 x 2 L). The combined organic layers were dried over $Na_2SO_4$ and concentrated. The residue was purified by flash column chromatography (gradient elution, 5-20% EtOAc in hexanes) to give (3S,5S)-5-((1S,3S)-1-azido-3-(4-methoxy-3-(3-methoxypropoxy)benzoyl)-4-methylpentyl)-3-isopropyldihydrofuran-2(3H)-one (32.4 g).

<u>Example 16:</u> Preparation of (2S,4S,5S,7S)-N-(3-amino-2,2-dimethyl-3-oxopropyl)-5-azido-4-hydroxy-2-isopropyl-7-(4-methoxy-3-(3-methoxypropoxy)benzoyl)-8-methylnonanamide

A mixture of (3S,5S)-5-((1S,3S)-1-azido-3-(4-methoxy-3-(3-methoxypropoxy)benzoyl)-4-methylpentyl)-3-isopropyldihydrofuran-2(3H)-one (29.0 g, 49 mmol), 3-amino-2,2-dimethylpropionamide (11.38 g, 98 mmol) and 2-hydroxypyridine (1.1 g) in triethylamine (30 mL) was refluxed for 24 hrs. Then, the cooled reaction mixture was poured into saturated $NaHCO_3$ (500 mL) and extracted with EtOAc (3 x 250 mL). Combined organic layers were washed with

saturated $NaHCO_3$ (500 mL) and saturated NaCl (500 mL), and dried over $Na_2SO_4$. The solvent was evaporated to obtain (2S,4S,5S,7S)-N-(3-amino-2,2-dimethyl-3-oxopropyl)-5-azido-4-hydroxy-2-isopropyl-7-(4-methoxy-3-(3-methoxypropoxy)benzoyl)-8-methylnonanamide as an oil (32.3 g).

Example 17: Preparation of aliskiren

(2S,4S,5S,7S)-N-(3-amino-2,2-dimethyl-3-oxopropyl)-5-azido-4-hydroxy-2-isopropyl-7-(4-methoxy-3-(3-methoxypropoxy)benzoyl)-8-methylnonanamide (30.1 g, 50.8 mmol) was dissolved in ethanol (600 mL) and conc. HCl (2 mL) and hydrogenated in the presence of 5% Pd/C (3.6 g) at ambient temperature and 4.0 bar. The reaction mixture was filtered and the catalyst was washed with ethanol (100 mL). The solvent was evaporated and the residue was dissolved in water (500 mL). pH was adjusted to 12 by addition of 1M NaOH, and the solution was extracted with tert.-butyl methyl ether (TBME) (4 x 300 mL). The combined organic phases were dried over $Na_2SO_4$ and the solvent was evaporated to afford aliskiren as viscous oil (22.5 g).

Example 18: Preparation of aliskiren glutaric acid salt

Aliskiren (1.6 g, 2.9 mmol) was dissolved in dichloromethane (10 mL), and glutaric acid (0.191 g, 1.45 mmol) in ethanol (1 mL) was added while stirring. The solution was stirred at room temperature for 24 h. Then, the solvent was evaporated in vacuum and the product was dried under vacuum at 30°C over night.
Mp = 71.5-75.8°C
XRPD: amorphous

Example 19: Preparation of aliskiren L-malic acid salt

Aliskiren (1.6 g, 2.9 mmol) was dissolved in dichloromethane (10 mL), and L-malic acid (0.194 g, 1.45 mmol) in ethanol (1 mL) was added while stirring. The solution was stirred at room temperature for 24 h. Then, solvent was evaporated in vacuum, and the product was dried under vacuum at 30°C over night.
Mp = 79.9-85°C
XRPD: amorphous

Example 20: Preparation of aliskiren D-mandelic acid salt

Aliskiren (1.6 g, 2.9 mmol) was dissolved in dichloromethane (10 mL), and D-mandelic acid (0.441 g, 2.9 mmol) in ethanol (1 mL) was added while stirring. The solution was stirred at room temperature for 24 h. Then, the solvent was evaporated in vacuum and the product was dried under vacuum at 30°C over night.
Mp = 56-60°C
XRPD: amorphous

Example 21: Preparation of aliskiren oxalic acid salt

Aliskiren (1.6 g, 2.9 mmol) was dissolved in dichloromethane (10 mL), and oxalic acid (0.130 g, 1.45 mmol) in ethanol (1 mL) was added while stirring. The solution was stirred at room temperature for 24 h. Then, the solvent was evaporated in vacuum and the product was dried under vacuum at 30°C over night.
Mp = 88-95°C
XRPD: amorphous

Example 22: Preparation of aliskiren glycolic acid salt

Aliskiren (1.6 g, 2.9 mmol) was dissolved in dichloromethane (10 mL), and glycolic acid (0.220 g, 2.9 mmol) in ethanol (1 mL) was added while stirring. The solution was stirred at room temperature for 24 h. Then, the solvent was evaporated in vacuum and the product was dried under vacuum at 30°C over night.
Mp = 45-50°C
XRPD: amorphous

Example 23: Preparation of aliskiren citric acid salt

Aliskiren (1.6 g, 2.9 mmol) was dissolved in dichloromethane (10 mL), and citric acid (0.278 g, 1.45 mmol) in ethanol (1 mL) was added while stirring. The solution was stirred at room temperature for 24 hrs. Then, the solvent was

evaporated in vacuum and the product was dried under vacuum at 30°C over night.
Mp = 78-85°C
XRPD: amorphous

**Claims**

1. Process for preparing a compound of formula (XVI)

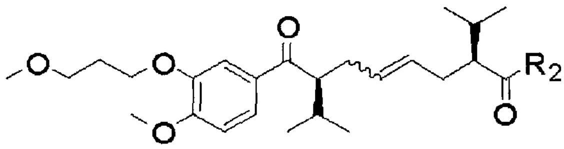

(XVI)

wherein $R_2$ is selected from the group consisting of halo, -OH, amino, alkyl, alkoxy, aryl, cycloalkyl, heterocyclyl or heteroaryl, wherein each of said amino, alkyl, alkoxy, aryl, cycloalkyl, heterocyclyl or heteroaryl can be unsubstituted or substituted,
comprising

(i3) reacting a compound of formula (XIII)

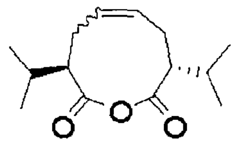

(XIII)

or a dimer thereof with a compound of formula (XXIII)

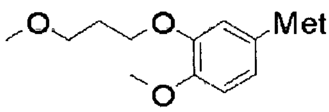

(XXIII)

wherein Met is a metal moiety,
to form a compound of formula (XVI).

2. Process according to claim 1, wherein the metal moiety Met is selected from -MgCl, -MgBr, -MgI, -ZnCl, -ZnBr, -ZnI or -Li, and preferably is -MgBr or -ZnCl.

3. Process according to claim 1 or 2, wherein $R_2$ is -Cl; a hydroxyl group; $C_1$-$C_6$ alkoxyl; alkylaryl, in particular -$(CH_2)_n$-aryl, wherein n is preferably ranging from 1 to 6 and aryl is preferably phenyl or naphthyl; (S)-4-$X_1$-oxazolidin-2-onyl such as (S)-4-benzyloxazolidin-2-onyl or (S)-4-phenyloxazolidin-2-onyl; or a N,O-dimethylhydroxylamino group.

**4.** Process according to any one of claims 1 to 3, wherein as compound of formula (XIII) the compound of formula (XIIIb) is used

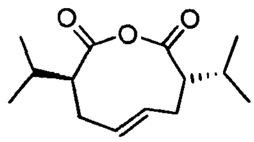

(XIIIb).

**5.** Process according to any one of claims 1 to 4, wherein the molar ratio of the compound of formula (XXIII) to the compound of formula (XIII) is 1:1 to 5:1, preferably 1:1 to 2:1 and more preferably 1.1:1 to 1.4:1.

**6.** Process according to any one of claims 1 to 5, wherein step (i3) is performed in an inert solvent selected from the group consisting of tetrahydrofuran, 2-methyltetrahydrofuran, diglyme, dioxan, diethyl ether, diisopropyl ether, tert-butyl methyl ether, cyclopentyl methyl ether, toluene and mixtures thereof, preferably in an inert solvent selected from tetrahydrofuran and/or toluene.

**7.** Process according to any one of claims 1 to 6, wherein step (i3) is performed at a temperature between -78°C to 35°C.

**8.** Process for preparing aliskiren of the following formula

(I) or a pharmaceutically acceptable salt thereof

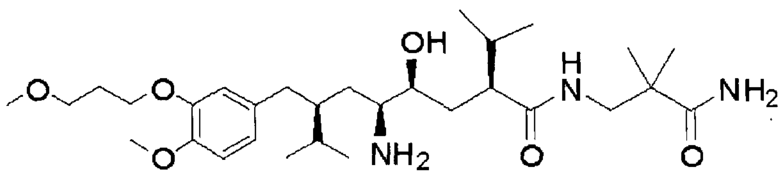

(I)

comprising
preparing a compound of formula (XVI) by a process according to any one of claims 1 to 7 and
converting the obtained compound of formula (XVI) into a compound of formula (I) or a pharmaceutically acceptable salt thereof.

**9.** Process according to claim 8, wherein the compound of formula (XVI) is converted into a compound of formula (I) or a pharmaceutically acceptable salt thereof by converting it into a compound of formula (XIX) and

(m) converting the compound of formula (XIX)

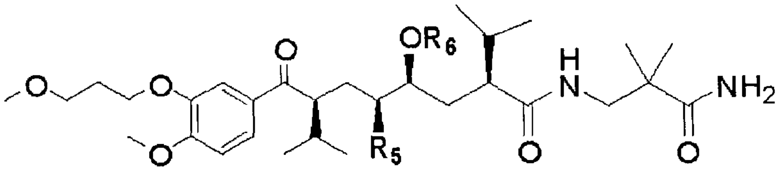

(XIX)

,

wherein
$R_5$ is $-N_3$ or $-NR_7R_B$, with $R_7$ and $R_8$ being independently selected from the group consisting of -H, alkyl, alkylaryl, aryloxyaryl, heterocyclyl, cycloalkyl, - C(O)-O-$R_9$ and -C(O)-$R_9$, wherein each of the alkyl, alkylaryl, aryloxyaryl, heterocyclyl and cycloalkyl can be unsubstituted or substituted, and wherein $R_9$ is selected from the group of consisting of alkyl, alkenyl, alkynyl, aryl and alkylaryl, with the proviso that if any one of $R_7$ and $R_8$ is -H, then the other of $R_7$ and $R_8$ is not -H or alkyl, or $R_7$ and $R_8$ combined together with the nitrogen atom to which they are attached form a 5 to 7 membered heterocyclic ring; and
$R_6$ is -H or a hydroxyl protecting group,
into the compound of formula (I) or a pharmaceutically acceptable salt thereof.

**10.** Process according to claim 9, wherein $R_5$ is $-N_3$ and/or $R_6$ is -H.

**11.** Process according to claim 9 or 10, wherein the compound of formula (XIX) is converted using a metal hydride or using hydrogen in the presence of a catalyst.

**12.** Process according to any one of claims 9 to 11, wherein the compound of formula (XIX) is converted in an organic solvent which is an aliphatic alcohol, preferably ethanol.

**13.** Process according to any one of claims 9 to 12, wherein the compound of formula (XIX) is converted in the presence of an acid catalyst, preferably HCl.

**14.** Process according to any one of claims 9 to 13, wherein the reaction temperature ranges from 10 to 50°C.

**15.** Process according to any one of claims 8 to 14, wherein the compound of formula (XVI) is converted to aliskiren by a method B which method comprises
converting the compound of formula (XVI) to compound (XVIII),

(1) reacting the compound of formula (XVIII)

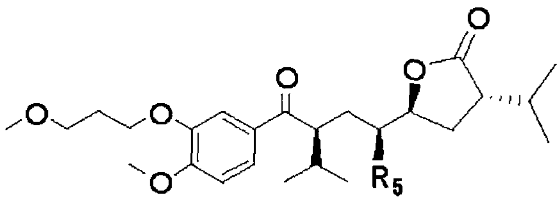

(XVIII)

,

wherein $R_5$ is defined as in claim 9, with an amine of formula (XIV)

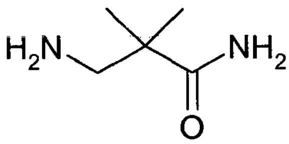

(XIV)

to obtain the compound of formula (XIX), and converting the compound of formula (XIX) to aliskiren.

**16.** Process according to claim 15, wherein the compound of formula (XVIII) is prepared by

(j1) lactonizing a compound of formula (XVI)

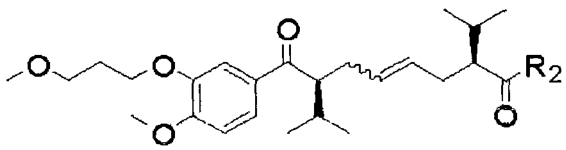

(XVI)

wherein $R_2$ is selected from the group consisting of halo, -OH, amino, alkyl, alkoxy, aryl, cycloalkyl, heterocyclyl or heteroaryl, wherein each of said amino, alkyl, alkoxy, aryl, cycloalkyl, heterocyclyl or heteroaryl can be unsubstituted or substituted,
to form a compound of formula (XVII)

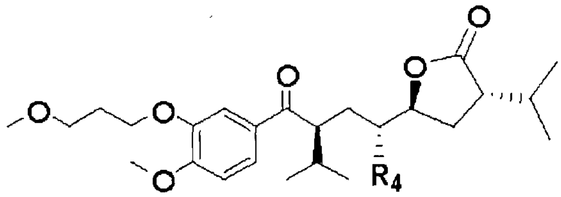

(XVII)

,

wherein $R_4$ is -Cl, -Br, -I or -OH, and
(k1) converting the compound of formula (XVII) to form the compound of formula (XVIII).

**17.** Process according to any one of claims 1 to 16, wherein the compound of formula (XIII) is prepared by

(g1) converting a compound of formula (VIII)

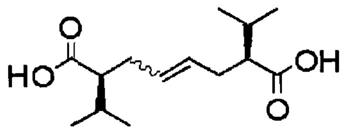

(VIII)

into the compound of formula (XIII);
or
(e2) converting a compound of formula (XII)

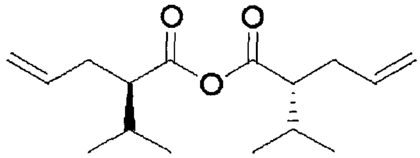

(XII)

into the compound of formula (XIII).

**18.** Process according to claim 17, wherein the compound of formula (VIII) is prepared by

(e2) converting a compound of formula (XII)

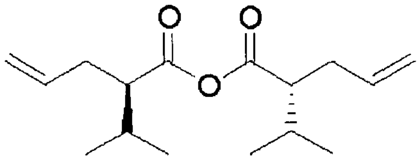

(XII)

into a mixture comprising the compounds of formula (XIII) and (XIIIa)

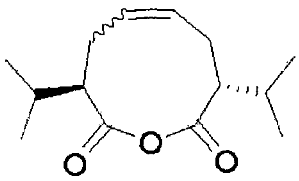

(XIII)

\+

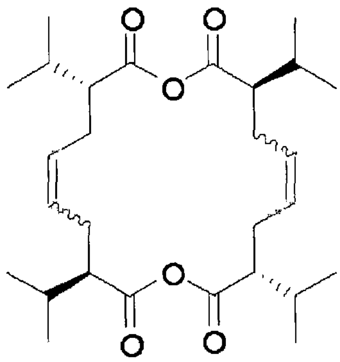

(XIIIa)

,

and

(h) converting the mixture comprising the compounds of formula (XIII) and (XIIIa) into the compound of formula (VIII);

or

(e3) converting a compound of formula (IV)

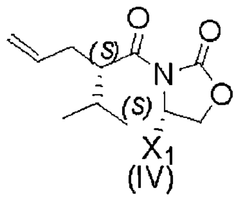

(IV)

wherein $X_1$ is an aryl or alkylaryl group, into a compound of formula (X)

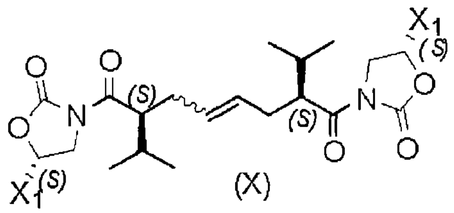

(X)

and

(c1) converting the compound of formula (X) into the compound of formula (VIII);

or

(e4) converting a compound of formula (V)

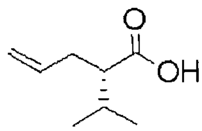

(V)

into the compound of formula (VIII);

or

(d) converting a compound of formula (V)

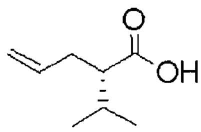

(V)

into a compound of formula (VI)

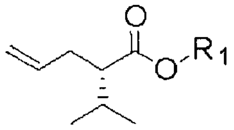

(VI)

wherein $R_1$ is an alkyl or alkylaryl group,
(e5) converting the compound of formula (VI) into a compound of formula (IX)

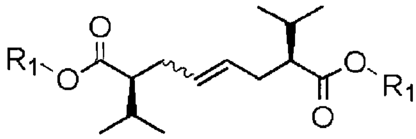

(IX)

wherein $R_1$ is an alkyl or alkylaryl group, and
(c2) converting the compound of formula (IX) into the compound of formula (VIII).

**19.** Process according to claim 18, wherein the compound of formula (XII) is prepared by

(g2) converting a compound of formula (V)

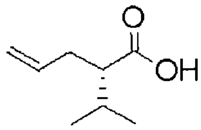

(V)

into the compound of formula (XII).

**20.** Process according to claim 18 or 19, wherein the compound of formula (V) is prepared by

(c3) converting a compound of formula (IV)

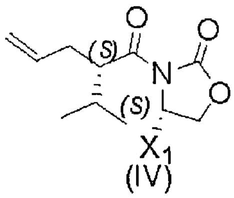

(IV)

wherein $X_1$ is an aryl or alkylaryl group,

into the compound of formula (V).

**21.** Process according to claim 18 or 20, wherein the compound of formula (IV) is prepared by

(a) converting a compound of formula (II)

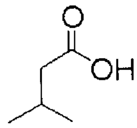

(II)

into a compound of formula (III)

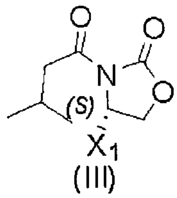

(III)

wherein $X_1$ is an aryl or alkylaryl group, and
(b) converting the compound of formula (III) into the compound of formula (IV).

**22.** Process for preparing aliskiren of the following formula (I) or a pharmaceutically acceptable salt thereof

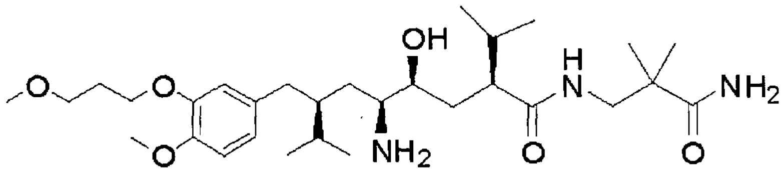

(I)

comprising step (i3) as defined in any one of claims 1 to 7 and at least one reaction step selected from the group consisting of step (e2), step (g1), step (h), step (j1), step (k1), step (1) and step (m), wherein each of said reaction steps is defined as in any one of claims 1 to 7 and 9 to 18 and wherein, with the exception of step (m), the product of each of said reaction steps is converted into a compound of formula (I).

**23.** A compound selected from

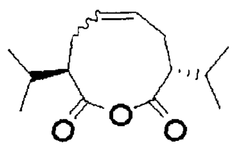

(XIII)

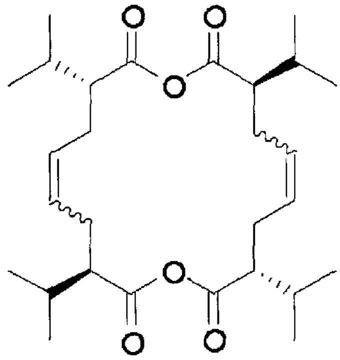

(XIIIa)

;

and

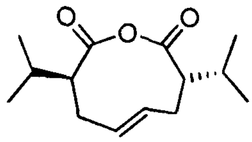

(XIIIb);

or a salt or solvate thereof.

**24.** Use of a compound according to claim 23 for preparing aliskiren of the following formula (I) or a pharmaceutically acceptable salt thereof

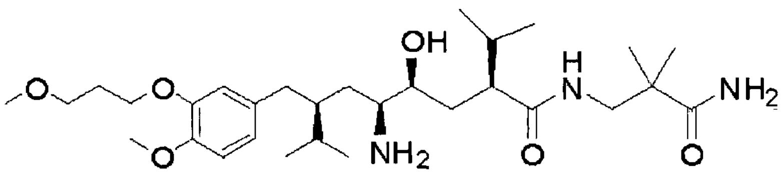

(I)

.

**25.** Process for preparing aliskiren of the following formula (I) or a pharmaceutically acceptable salt thereof

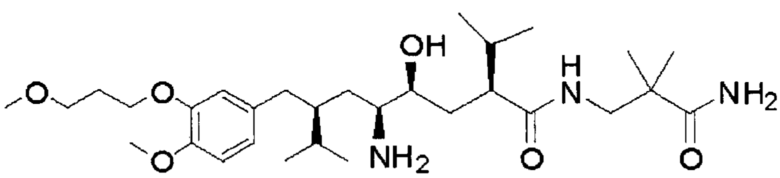

(I)

which comprises
providing a compound according to claim 23, and converting said compound to aliskiren.

**26.** Process for preparing a pharmaceutical composition containing aliskiren or a pharmaceutically acceptable salt thereof, comprising preparing aliskiren or a pharmaceutically acceptable salt thereof in accordance with a process of any one of claims 8 to 22 or 25 and converting the aliskiren or salt thereof into the pharmaceutical composition, preferably by a hot-melt and solvent-free granulation process.

## Patentansprüche

**1.** Verfahren zur Herstellung einer Verbindung der Formel (XVI)

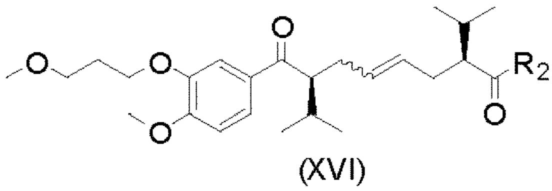

(XVI)

wobei $R_2$ aus der Gruppe bestehend aus Halogen, -OH, Amino, Alkyl, Alkoxy, Aryl, Cycloalkyl, Heterocyclyl oder Heteroaryl ausgewählt ist, wobei jedes der Amino, Alkyl, Alkoxy, Aryl, Cycloalkyl, Heterocyclyl oder Heteroaryl unsubstituiert oder substituiert sein kann,
bei dem

(i3) eine Verbindung der Formel (XIII)

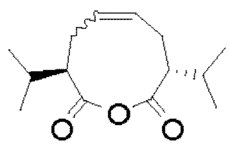

(XIII)

oder ein Dimer davon mit einer Verbindung der Formel (XXIII),

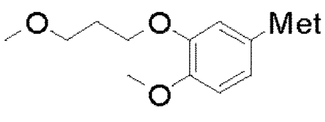

(XXIII) ,

wobei Met ein Metallrest ist,
umgesetzt wird, um eine Verbindung der Formel (XVI) zu bilden.

**2.** Verfahren nach Anspruch 1, bei dem der Metallrest Met ausgewählt ist aus -MgCl, -MgBr, -MgI, -ZnCl, -ZnBr, -ZnI oder -Li und vorzugsweise -MgBr oder -ZnCl ist.

**3.** Verfahren nach Anspruch 1 oder 2, bei dem $R_2$ -Cl, eine Hydroxygruppe, $C_1$-$C_6$-Alkoxy, Alkylaryl, vorzugsweise $-(CH_2)_n$-Aryl, wobei n vorzugsweise zwischen 1 und 6 ist und Aryl vorzugsweise Phenyl oder Naphthyl ist, (S)-4-$X_1$-Oxazolidin-2-onyl wie z.B. (S)-4-Benzyloxazolidin-2-onyl oder (S)-4-Phenyloxazolidin-2-onyl, oder eine N,O-Dimethylhydroxyaminogruppe ist.

4. Verfahren nach einem der Ansprüche 1 bis 3, bei dem als Verbindung der Formel (XIII) die Verbindung der Formel (XIIIb) verwendet wird

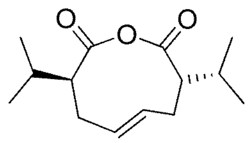

(XIIIb).

5. Verfahren nach einem der Ansprüche 1 bis 4, bei dem das Molverhältnis der Verbindung der Formel (XXIII) zu der Verbindung der Formel (XIII) 1:1 bis 5:1, vorzugsweise 1:1 bis 2:1, und insbesondere 1,1:1 bis 1,4:1 ist.

6. Verfahren nach einem der Ansprüche 1 bis 5, bei dem Schritt (i3) in einem inerten Lösungsmittel ausgewählt aus der Gruppe bestehend aus Tetrahydrofuran, 2-Methyltetrahydrofuran, Diglyme, Dioxan, Diethylether, Diisopropylether, tert-Butylmethylether, Cyclopentylmethylether, Toluol und Mischungen davon ausgeführt wird, vorzugsweise in einem inerten Lösungsmittel ausgewählt aus Tetrahydrofuran und/oder Toluol.

7. Verfahren nach einem der Ansprüche 1 bis 6, bei dem Schritt (i3) bei einer Temperatur zwischen -78°C und 35°C durchgeführt wird.

8. Verfahren zur Herstellung von Aliskiren der folgenden Formel (I) oder eines pharmazeutisch annehmbaren Salzes davon

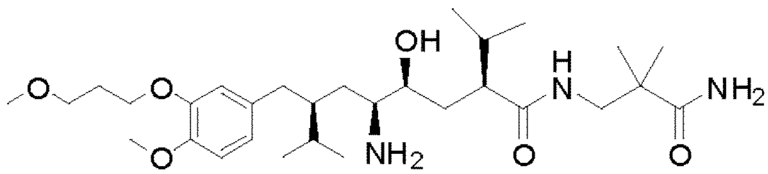

(I)

,

bei dem
eine Verbindung der Formel (XVI) in einem Verfahren nach einem der Ansprüche 1 bis 7 hergestellt wird und
die erhaltene Verbindung der Formel (XVI) in eine Verbindung der Formel (I) oder ein pharmazeutisch annehmbares Salz davon umgewandelt wird.

9. Verfahren nach Anspruch 8, bei dem die Verbindung der Formel (XVI) in eine Verbindung der Formel (I) oder ein pharmazeutisch annehmbares Salz davon umgewandelt wird, indem sie in eine Verbindung der Formel (XIX) umgewandelt wird und
(m) die Verbindung der Formel (XIX)

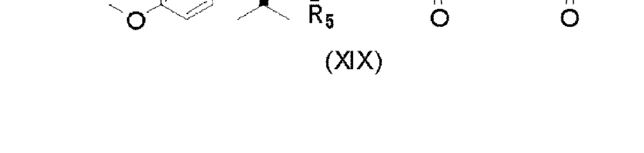

(XIX)

,

wobei $R_5$ $-N_3$ oder $-NR_7R_8$ ist und $R_7$ und $R_8$ unabhängig aus der Gruppe bestehend aus -H, Alkyl, Alkylaryl, Aryloxyaryl, Heterocyclyl, Cycloalkyl, $-C(O)-O-R_9$ und $-C(O)-R_9$ ausgewählt sind, wobei jedes der Alkyl, Alkylaryl, Aryloxyaryl, Heterocyclyl und Cycloalkyl unsubstituiert oder substituiert sein kann und wobei $R_9$ aus der Gruppe bestehend aus Alkyl, Alkenyl, Alkinyl, Aryl und Alkylaryl ausgewählt ist, mit der Bedingung, dass, falls einer von $R_7$ und $R_8$ -H ist, der andere $R_7$ und $R_8$ nicht -H oder Alkyl ist oder $R_7$ und $R_8$ zusammen mit dem Stickstoffatom, an das sie gebunden sind, einen 5 bis 7-gliedrigen Heterozyklus bilden und
$R_6$ ein -H oder eine Hydroxy-Schutzgruppe ist,
in eine Verbindung der Formel (I) oder ein pharmazeutisch annehmbares Salz davon umgewandelt wird.

**10.** Verfahren nach Anspruch 9, bei dem $R_5$ $-N_3$ ist und/oder $R_6$ - H ist.

**11.** Verfahren nach Anspruch 9 oder 10, bei dem die Verbindung der Formel (XIX) unter Verwendung eines Metallhydrids oder unter Verwendung von Wasserstoff in Gegenwart eines Katalysators umgewandelt wird.

**12.** Verfahren nach einem der Ansprüche 9 bis 11, bei dem die Verbindung der Formel (XIX) in einem organischen Lösungsmittel umgewandelt wird, das ein aliphatischer Alkohol, vorzugsweise Ethanol, ist.

**13.** Verfahren nach einem der Ansprüche 9 bis 12, bei dem die Verbindung der Formel (XIX) in Gegenwart eines sauren Katalysators, vorzugsweise HCl, umgewandelt wird.

**14.** Verfahren nach einem der Ansprüche 9 bis 13, bei dem die Reaktionstemperatur zwischen 10 und 50°C liegt.

**15.** Verfahren nach einem der Ansprüche 8 bis 14, bei dem die Verbindung der Formel (XVI) durch ein Verfahren B in Aliskiren umgewandelt wird, bei dem
die Verbindung der Formel (XVI) in Verbindung (XVIII) umgewandelt wird,

(1) die Verbindung der Formel (XVIII)

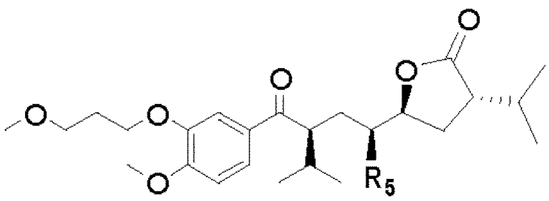

(XVIII)

,

wobei $R_5$ wie in Anspruch 9 definiert ist, mit einem Amin der Formel (XIV)

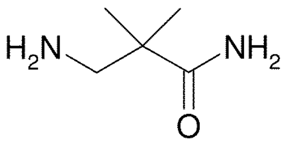

(XIV)

umgesetzt wird, um die Verbindung der Formel (XIX) zu erhalten und die Verbindung der Formel (XIX) in Aliskiren umgewandelt wird.

**16.** Verfahren nach Anspruch 15, bei dem die Verbindung der Formel (XVIII) hergestellt wird, indem

(j1) eine Verbindung der Formel (XVI)

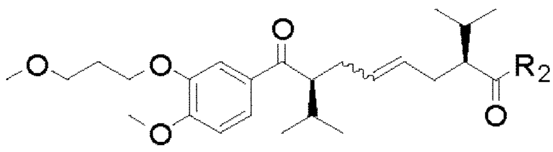

(XVI)

,

laktonisiert wird, wobei $R_2$ ausgewählt ist aus der Gruppe bestehend aus Halogen, -OH, Amino, Alkyl, Alkoxy, Aryl, Cycloalkyl, Heterocyclyl oder Heteroaryl, wobei jedes der Amino, Alkyl, Alkoxy, Aryl, Cycloalkyl, Heterocyclyl, oder Heteroaryl unsubstituiert oder substuiert sein kann, um eine Verbindung der Formel (XVII)

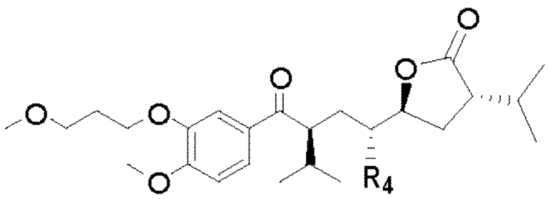

(XVII)

,

zu bilden, wobei $R_4$ -Cl, -Br, -I oder -OH ist, und (k1) die Verbindung der Formel (XVII) unter Bildung der Verbindung der Formel (XVIII) umgewandelt wird.

**17.** Verfahren nach einem der Ansprüche 1 bis 16, bei dem die Verbindung der Formel (XIII) hergestellt wird, indem

(g1) eine Verbindung der Formel (VIII)

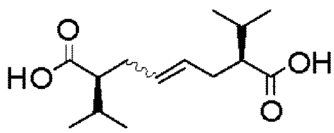

(VIII)

in die Verbindung der Formel (XIII) umgewandelt wird oder (e2) eine Verbindung der Formel (XII)

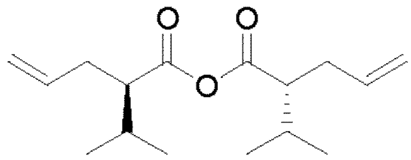

(XII)

in die Verbindung der Formel (XIII) umgewandelt wird.

**18.** Verfahren nach Anspruch 17, bei dem die Verbindung der Formel (VIII) hergestellt wird, indem

(e2) eine Verbindung der Formel (XII)

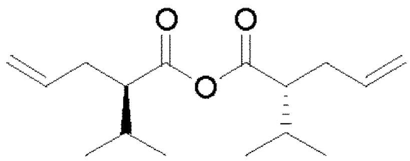

(XII)

in eine Mischung umgewandelt wird, die die Verbindungen der Formeln (XIII) und (XIIIa)

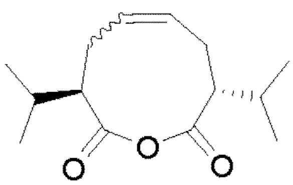

(XIII)

\+

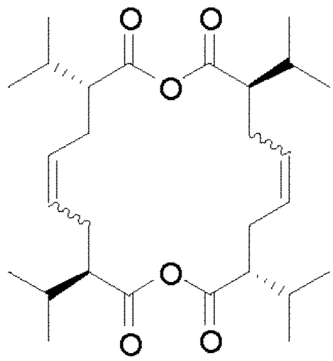

(XIIIa)

umfasst, und
(h) die Mischung, die die Verbindungen der Formeln (XIII) und (XIIIa) umfasst, in die Verbindung der Formel (VIII) umgewandelt wird,
oder indem
(e3) eine Verbindung der Formel (IV)

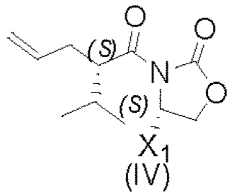

(IV)

,

wobei $X_1$ eine Aryl- oder Alkylarylgruppe ist, in eine Verbindung der Formel (X)

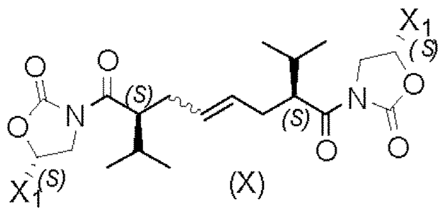

(X)

umgewandelt wird und
(c1) die Verbindung der Formel (X) in die Verbindung der Formel (VIII) umgewandelt wird,
oder indem
(e4) die Verbindung der Formel (V)

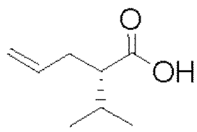

(V)

in die Verbindung der Formel (VIII) umgewandelt wird oder indem
(d) die Verbindung der Formel (V)

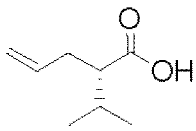

(V)

in eine Verbindung der Formel (VI)

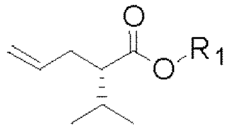

(VI)

umgewandelt wird, wobei $R_1$ eine Alkyl- oder Alkylarylgruppe ist,
(e5) die Verbindung der Formel (VI) in eine Verbindung der Formel (IX)

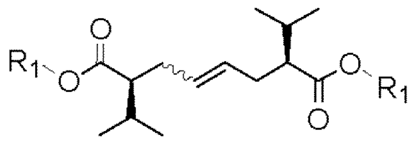

(IX)

,

umgewandelt wird, wobei $R_1$ eine Alkyl- oder Alkylarylgruppe ist, und
(c2) die Verbindung der Formel (IX) in die Verbindung der Formel (VIII) umgewandelt wird.

**19.** Verfahren nach Anspruch 18, bei dem die Verbindung der Formel (XII) hergestellt wird, indem

(g2) eine Verbindung der Formel (V)

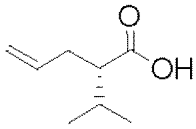

(V)

in die Verbindung der Formel (XII) umgewandelt wird.

**20.** Verfahren nach Anspruch 18 oder 19, bei dem die Verbindung der Formel (V) hergestellt wird, indem

(c3) eine Verbindung der Formel (IV)

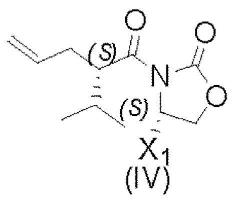

(IV)

,

wobei $X_1$ eine Aryl- oder Alkylarylgruppe ist, in die Verbindung der Formel (V) umgewandelt wird.

**21.** Verfahren nach Anspruch 18 oder 20, bei dem die Verbindung der Formel (IV) hergestellt wird, indem

(a) eine Verbindung der Formel (II)

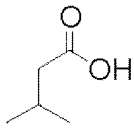
(II)

in eine Verbindung der Formel (III)

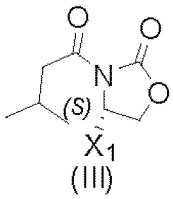
(III)

umgewandelt wird, wobei $X_1$ eine Aryl- oder Alkylarylgruppe ist, und
(b) die Verbindung der Formel (III) in die Verbindung der Formel (IV) umgewandelt wird.

**22.** Verfahren zur Herstellung von Aliskiren der folgenden Formel (I) oder eines pharmazeutisch annehmbaren Salzes davon

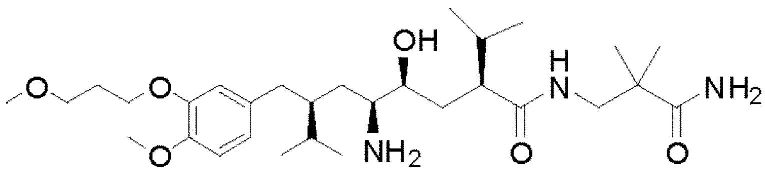
(I)

,

das Schritt (i3) wie in einem der Ansprüche 1 bis 7 definiert und mindestens einen Reaktionsschritt ausgewählt aus der Gruppe bestehend aus Schritt (e2), Schritt (g1), Schritt (h), Schritt (j1), Schritt (k1), Schritt (1) und Schritt (m) umfasst,
wobei jeder der Reaktionsschritte wie in einem der Ansprüche 1 bis 7 und 9 bis 18 definiert ist und
wobei mit Ausnahme von Schritt (m) das Produkt von jedem Reaktionsschritt in eine Verbindung der Formel (I) umgewandelt wird.

**23.** Eine Verbindung ausgewählt aus

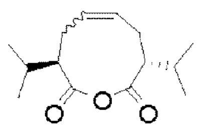

(XIII)

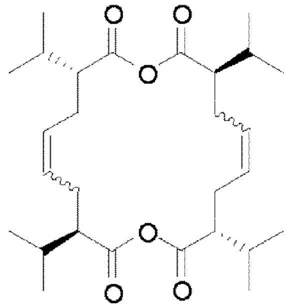

(XIIIa)

;

und

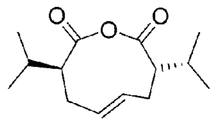

(XIIIb);

oder ein Salz oder ein Solvat davon.

**24.** Verwendung einer Verbindung gemäß Anspruch 23 zur Herstellung von Aliskiren der folgenden Formel (I) oder eines pharmazeutisch annehmbaren Salzes davon

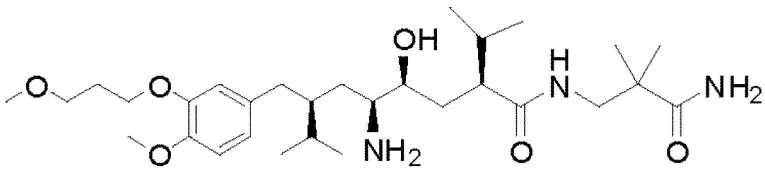

(I)

.

**25.** Verfahren zur Herstellung von Aliskiren der folgenden Formel (I) oder eines pharmazeutisch annehmbaren Salzes davon

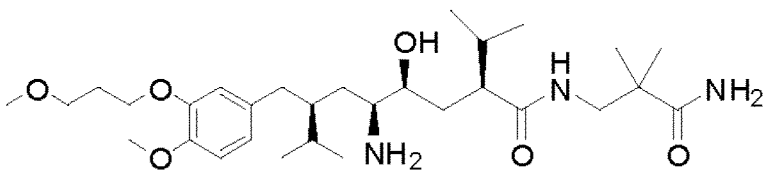

(I)

,

bei dem eine Verbindung gemäß Anspruch 23 bereitgestellt wird und die Verbindung in Aliskiren umgewandelt wird.

**26.** Verfahren zur Herstellung einer pharmazeutischen Zusammensetzung, die Aliskiren oder ein pharmazeutisch annehmbares Salz davon enthält, bei dem Aliskiren oder ein pharmazeutisch annehmbares Salz davon gemäß einem der Ansprüche 8 bis 22 oder 25 hergestellt wird und das Aliskiren oder Salz davon in die pharmazeutische Zusammensetzung überführt wird, vorzugsweise in einem Hot-Melt- oder lösemittelfreien Granulierungsverfahren.

**Revendications**

**1.** Procédé de préparation d'un composé de formule (XVI) :

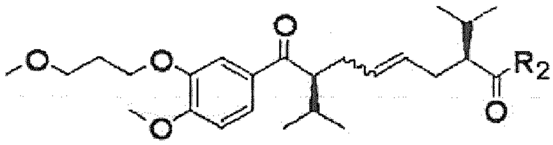

(XVI)

dans laquelle $R_2$ représente une entité choisie dans l'ensemble constitué par les atomes d'halogène et les groupes hydroxyle, amino, alkyle, alcoxy, aryle, cycloalkyle, hétérocyclyle ou hétéroaryle, étant entendu que chacun de ces groupes amino, alkyle, alcoxy, aryle, cycloalkyle, hétérocyclyle ou hétéroaryle peut être doté ou non de substituant(s), lequel procédé comporte :

(i3) le fait de faire réagir un composé de formule (XIII) :

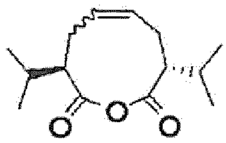

(XIII)

ou un dimère de ce composé,
avec un composé de formule (XXIII) :

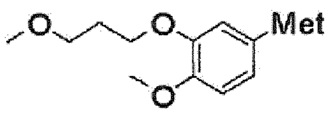

(XXIII)

dans laquelle Met représente une entité de nature métallique,
de manière à obtenir un composé de formule (XVI).

**2.** Procédé conforme à la revendication 1, dans lequel l'entité de nature métallique symbolisée par Met est choisie parmi les fragments de formule -MgCl, -MgBr, -MgI, -ZnCl, -ZnBr ou -ZnI et un atome de lithium, et de préférence, est un fragment de formule -MgBr ou -ZnCl.

**3.** Procédé conforme à la revendication 1 ou 2, dans lequel $R_2$ représente :

- un atome de chlore,
- un groupe hydroxyle,
- un groupe alcoxy en $C_1$-$C_6$,
- un groupe aryl-alkyle, et en particulier, un groupe symbolisé par $-(CH_2)_n$-Aryl où l'indice n vaut de préférence de 1 à 6 et "Aryl" représente de préférence un groupe phényle ou naphtyle,
- un groupe de type (S)-4-$X_1$-2-oxo-oxazolidinyle, comme un groupe (S)-4-benzyl-2-oxo-oxazolidinyle ou (S)-4-phényl-2-oxo-oxazolidinyle,
- ou un groupe N,O-diméthyl-hydroxylamino.

**4.** Procédé conforme à l'une des revendications 1 à 3, dans lequel on utilise, en tant que composé de formule (XIII), le composé de formule (XIIIb) :

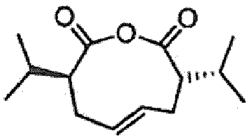

(XIIIb).

**5.** Procédé conforme à l'une des revendications 1 à 4, dans lequel le rapport molaire du composé de formule (XXIII) au composé de formule (XIII) vaut de 1/1 à 5/1, de préférence de 1/1 à 2/1, et mieux encore de 1,1/1 à 1,4/1.

**6.** Procédé conforme à l'une des revendications 1 à 5, dans lequel l'étape (i3) est réalisée dans un solvant inerte choisi dans l'ensemble constitué par les tétrahydrofurane, 2-méthyl-tétrahydrofurane, diglyme, dioxane, diéthyl-éther, diisopropyl-éther, tertiobutyl-méthyl-éther, cyclopentyl-méthyl-éther et toluène, ainsi que leurs mélanges, et de préférence dans un solvant inerte choisi parmi du tétrahydrofurane et/ou du toluène.

**7.** Procédé conforme à l'une des revendications 1 à 6, dans lequel l'étape (i3) est réalisée à une température valant de -78 °C à 35 °C.

**8.** Procédé de préparation de l'aliskirène, de formule (I) suivante, ou d'un sel pharmacologiquement admissible de ce composé :

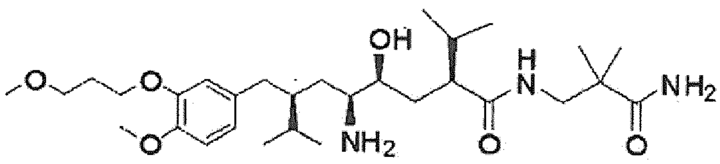

(I)

lequel procédé comporte les étapes suivantes :

- préparer un composé de formule (XVI), en opérant selon un procédé conforme à l'une des revendications 1 à 7,
- et convertir le composé de formule (XVI) ainsi obtenu en le composé de formule (I) ou en un sel pharmacologiquement admissible de ce composé.

**9.** Procédé conforme à la revendication 8, dans lequel on convertit un composé de formule (XVI) en le composé de formule (I) ou en un sel pharmacologiquement admissible de ce composé en le convertissant en un composé de formule (XIX),

(m) et en convertissant ce composé de formule (XIX) :

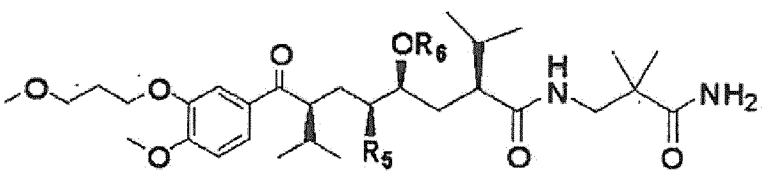

(XIX)

dans laquelle
$R_5$ représente un groupe de formule $-N_3$ ou $-NR_7R_8$, où $R_7$ et $R_8$ représentent des entités choisies, indépendamment, dans l'ensemble constitué par un atome d'hydrogène, les groupes alkyle, aryl-alkyle, aryl-oxy-aryle, hétérocyclyle et cycloalkyle, et les groupes de formule $-C(O)-O-R_9$ ou $-C(O)-R_9$, étant entendu que chacun de ces groupes alkyle, aryl-alkyle, aryl-oxy-aryle, hétérocyclyle ou cycloalkyle peut être doté ou non de substituant(s), et que $R_9$ représente une entité choisie dans l'ensemble constitué par les groupes alkyle, alcényle,

alcynyle, aryle et aryl-alkyle, sous réserve que, si l'un des symboles $R_7$ et $R_8$ représente un atome d'hydrogène, l'autre de ces symboles $R_7$ et $R_8$ ne représente pas un atome d'hydrogène ou un groupe alkyle, ou bien $R_7$ et $R_8$ représentent des entités qui forment, conjointement avec l'atome d'azote auquel elles sont liées, un hétérocycle comportant de 5 à 7 chaînons,
et $R_6$ représente un atome d'hydrogène ou un groupe hydroxy-protecteur,
en le composé de formule (I) ou en un sel pharmacologiquement admissible de ce composé.

**10.** Procédé conforme à la revendication 9, dans lequel $R_5$ représente un groupe de formule $-N_3$ et/ou $R_6$ représente un atome d'hydrogène.

**11.** Procédé conforme à la revendication 9 ou 10, dans lequel la conversion du composé de formule (XIX) est réalisée au moyen d'un hydrure de métal, ou au moyen d'hydrogène en présence d'un catalyseur.

**12.** Procédé conforme à l'une des revendications 9 à 11, dans lequel la conversion du composé de formule (XIX) est réalisée dans un solvant organique qui est un alcool aliphatique, et de préférence de l'éthanol.

**13.** Procédé conforme à l'une des revendications 9 à 12, dans lequel la conversion du composé de formule (XIX) est réalisée en présence d'un catalyseur acide, qui est de préférence du chlorure d'hydrogène.

**14.** Procédé conforme à l'une des revendications 9 à 13, dans lequel la température de réaction vaut de 10 à 50 °C.

**15.** Procédé conforme à l'une des revendications 8 à 14, dans lequel on convertit le composé de formule (XVI) en aliskirène en opérant selon un procédé B, lequel procédé comporte les étapes suivantes :

- convertir le composé de formule (XVI) en un composé de formule (XVIII),
- (1) faire réagir ce composé de formule (XVIII) :

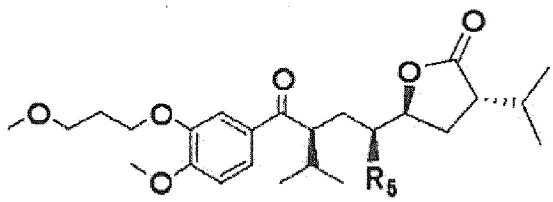

(XVIII)

dans laquelle le symbole $R_5$ a la signification indiquée dans la revendication 9,
avec une amine de formule (XIV) :

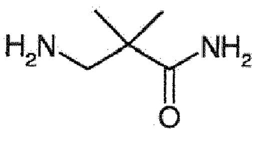

(XIV)

de manière à obtenir un composé de formule (XIX),
- et convertir ce composé de formule (XIX) en aliskirène.

**16.** Procédé conforme à la revendication 15, dans lequel on prépare le composé de formule (XVIII) en opérant comme suit :

(j1) on effectue la lactonisation d'un composé de formule (XVI) :

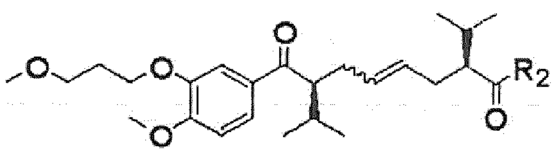

(XVI)

dans laquelle $R_2$ représente une entité choisie dans l'ensemble constitué par les atomes d'halogène et les groupes hydroxyle, amino, alkyle, alcoxy, aryle, cycloalkyle, hétérocyclyle ou hétéroaryle, étant entendu que chacun de ces groupes amino, alkyle, alcoxy, aryle, cycloalkyle, hétérocyclyle ou hétéroaryle peut être doté ou non de substituant(s),
de manière à obtenir un composé de formule (XVII) :

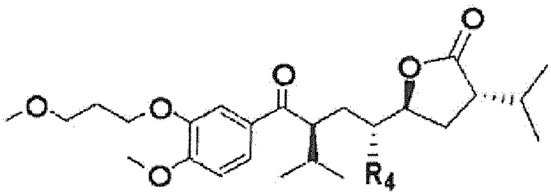

(XVII)

dans laquelle $R_4$ représente un atome de chlore, de brome ou d'iode ou un groupe hydroxyle,
(k1) et l'on convertit ce composé de formule (XVII) de manière à obtenir un composé de formule (XVIII).

**17.** Procédé conforme à l'une des revendications 1 à 16, pour lequel on prépare le composé de formule (XIII) en opérant comme suit :

(g1) on convertit le composé de formule (VIII) :

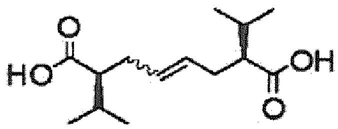

(VIII)

en le composé de formule (XIII),
(e2) ou l'on convertit le composé de formule (XII) :

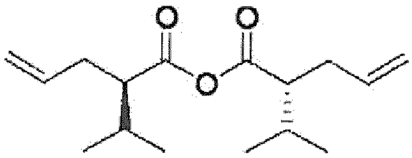

(XII)

en le composé de formule (XIII).

**18.** Procédé conforme à la revendication 17, pour lequel on prépare le composé de formule (VIII) en opérant comme suit :

(e2) on convertit le composé de formule (XII) :

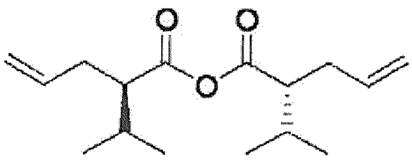

(XII)

en un mélange comprenant des composés de formules (XIII) et (XIIIa) :

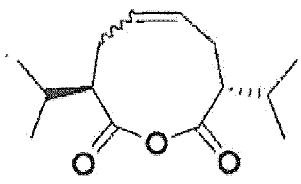

(XIII)

+

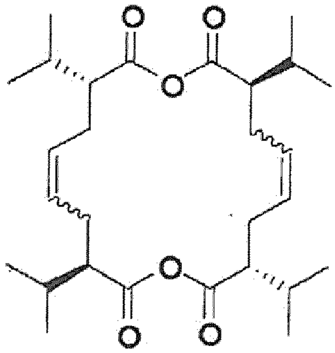

(XIIIa)

(h) et l'on convertit ce mélange comprenant des composés de formules (XIII) et (XIIIa) en un composé de formule (VIII) ;

ou bien

(e3) on convertit un composé de formule (IV) :

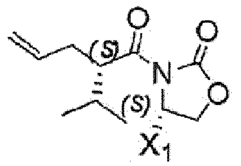

(IV)

dans laquelle $X_1$ représente un groupe aryle ou aryl-alkyle, en un composé de formule (X) :

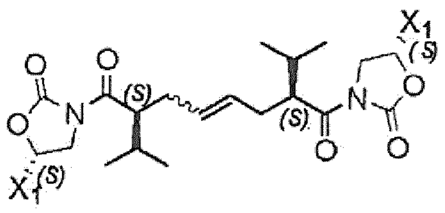

(X)

(c1) et l'on convertit ce composé de formule (X) en un composé de formule (VIII) ;

ou bien

(e4) on convertit le composé de formule (V) :

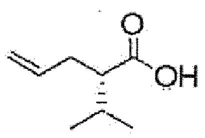

(V)

en un composé de formule (VIII) ;

ou bien

(d) on convertit le composé de formule (V) :

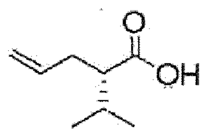

(V)

en un composé de formule (VI) :

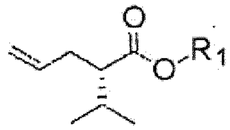

(VI)

dans laquelle $R_1$ représente un groupe aryle ou aryl-alkyle,
(e5) on convertit ce composé de formule (VI) en un composé de formule (IX) :

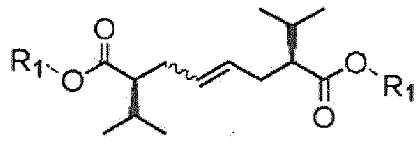

(IX)

dans laquelle $R_1$ représente un groupe aryle ou aryl-alkyle,
(e4) et l'on convertit ce composé de formule (IX) en un composé de formule (VIII).

**19.** Procédé conforme à la revendication 18, pour lequel on prépare le composé de formule (XII) en opérant comme suit :

(g2) on convertit le composé de formule (V) :

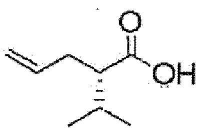

(V)

en le composé de formule (XII).

**20.** Procédé conforme à la revendication 18 ou 19, pour lequel on prépare le composé de formule (V) en opérant comme suit :

(c3) on convertit un composé de formule (IV) :

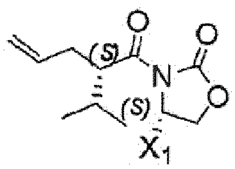

(IV)

dans laquelle $X_1$ représente un groupe aryle ou aryl-alkyle, en le composé de formule (V).

**21.** Procédé conforme à la revendication 18 ou 20, pour lequel on prépare le composé de formule (IV) en opérant comme suit :

(a) on convertit le composé de formule (II) :

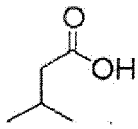

(II)

en un composé de formule (III) :

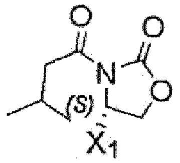

(III)

dans laquelle $X_1$ représente un groupe aryle ou aryl-alkyle,
(b) et l'on convertit ce composé de formule (III) en un composé de formule (IV).

**22.** Procédé de préparation de l'aliskirène, de formule (I) suivante, ou d'un sel pharmacologiquement admissible de ce composé :

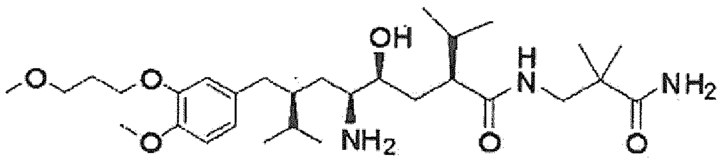

(I)

lequel procédé comporte une étape (i3), définie dans l'une des revendications 1 à 7, et au moins une étape de réaction choisie dans l'ensemble formé par l'étape (e2), l'étape (g1), l'étape (h), l'étape (j1), l'étape (k1), l'étape (1), et l'étape (m), chacune de ces étapes de réaction étant définie dans l'une des revendications 7 et 9 à 18,
et dans lequel procédé, excepté pour l'étape (m), le produit de chacune de ces étapes de réaction est converti en le composé de formule (I).

**23.** Composé choisi parmi ceux de formules suivantes :

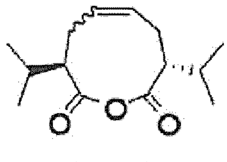

(XIII)

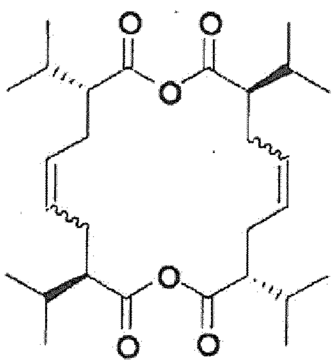

(XIIIa)

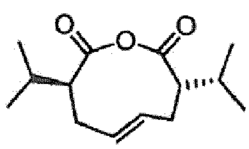

(XIIIb)

ou sel ou solvat d'un tel composé.

**24.** Utilisation d'un composé conforme à la revendication 23 en vue de la préparation de l'aliskirène, de formule (I) suivante, ou d'un sel pharmacologiquement admissible de ce composé :

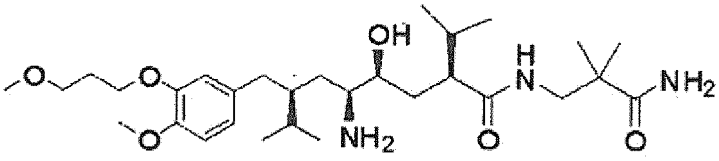

(I).

**25.** Procédé de préparation de l'aliskirène, de formule (I) suivante, ou d'un sel pharmacologiquement admissible de ce composé :

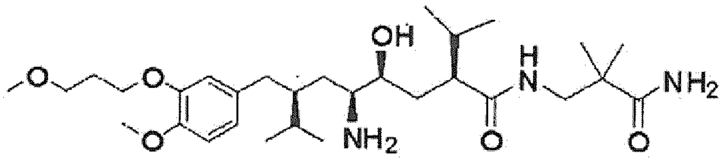

(I)

lequel procédé comporte les étapes suivantes :

- prendre un composé conforme à la revendication 23,
- et convertir ce composé en aliskirène.

**26.** Procédé de préparation d'une composition pharmaceutique contenant de l'aliskirène ou un sel pharmacologiquement admissible de ce composé, lequel procédé comporte le fait de préparer de l'aliskirène ou un sel pharmacologiquement admissible de ce composé, en opérant selon un procédé conforme à l'une des revendications 8 à 22 et 25, et le fait de mettre cet aliskirène ou ce sel sous la forme d'une composition pharmaceutique, de préférence selon un procédé de granulation à chaud et sans solvant.

Figure 1

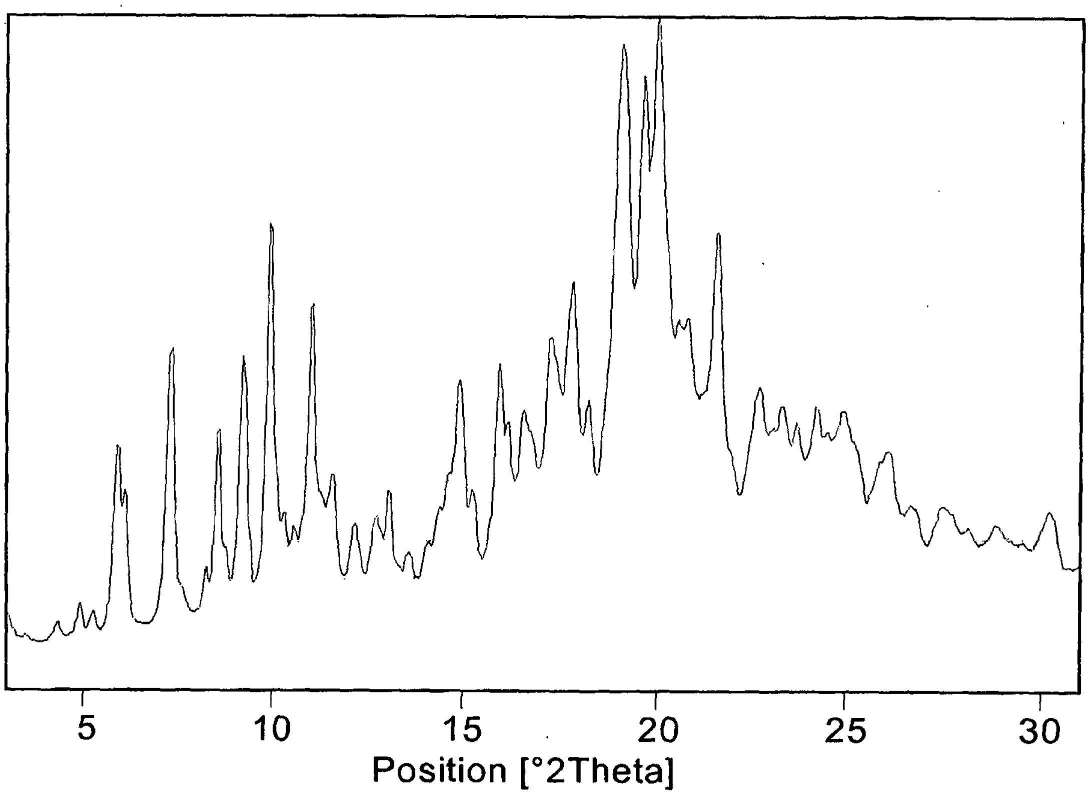

Figure 2

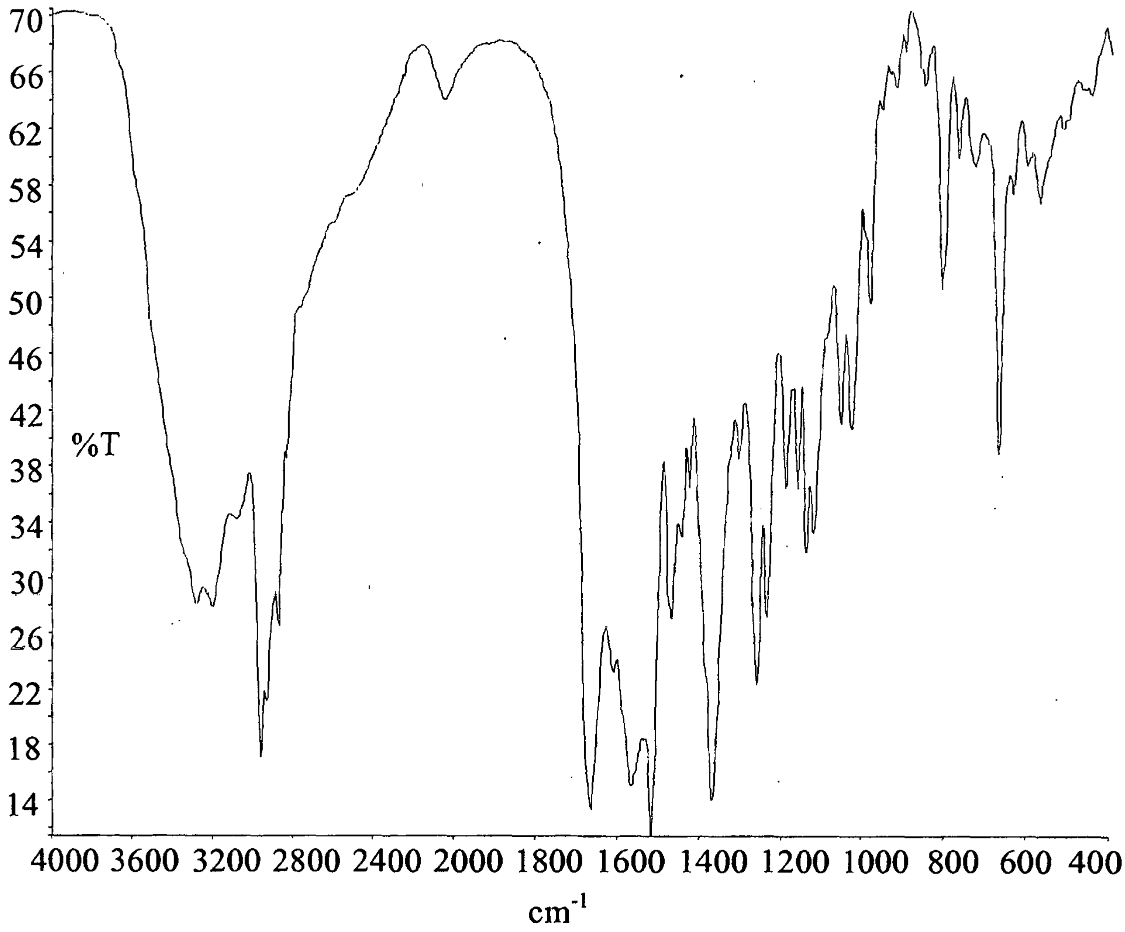
70
66
62
58
54
50
46
42
%T
38
34
30
26
22
18
14
4000 3600 3200 2800 2400 2000 1800 1600 1400 1200 1000 800 600 400
cm-1

Figure 3

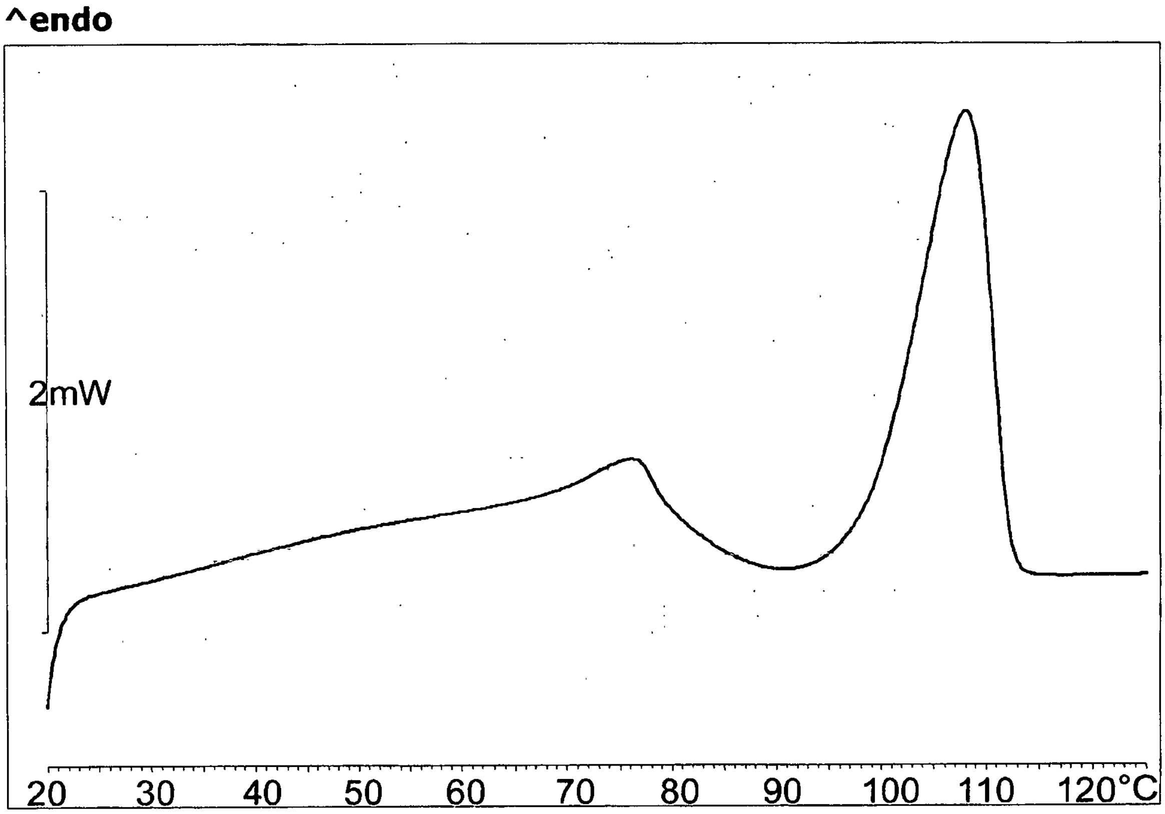

Figure 4

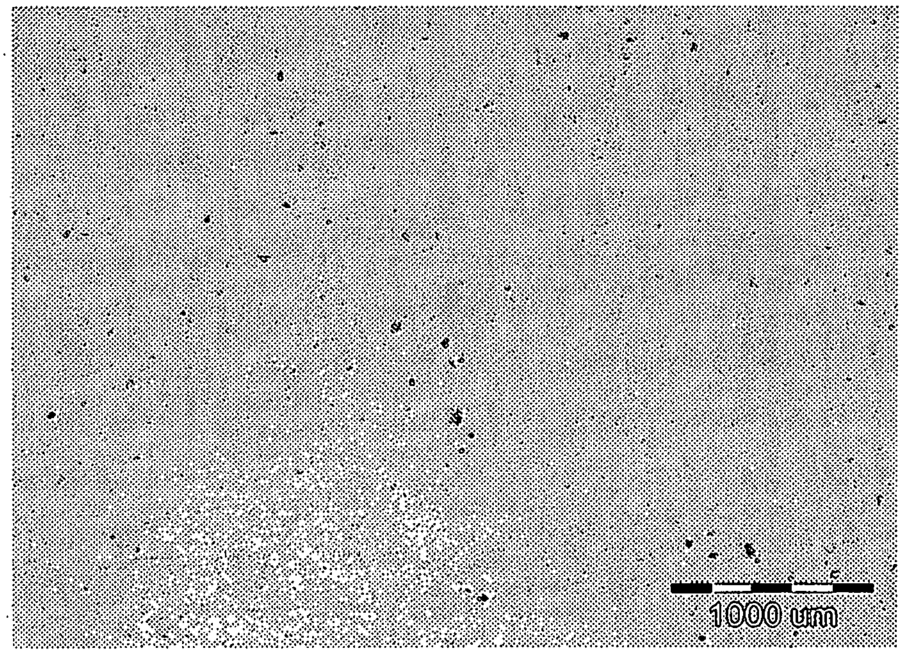

Figure 5

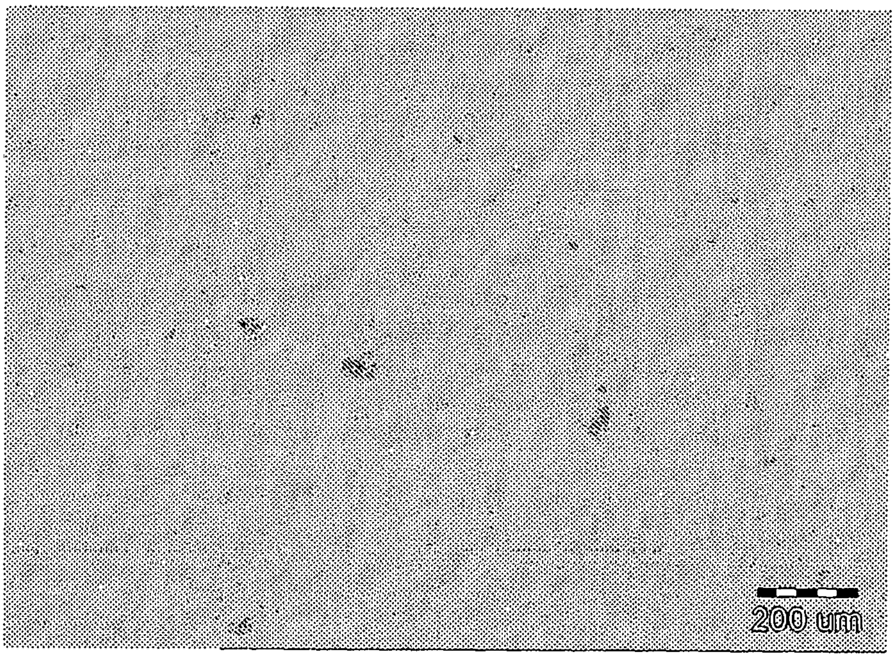

Figure 6

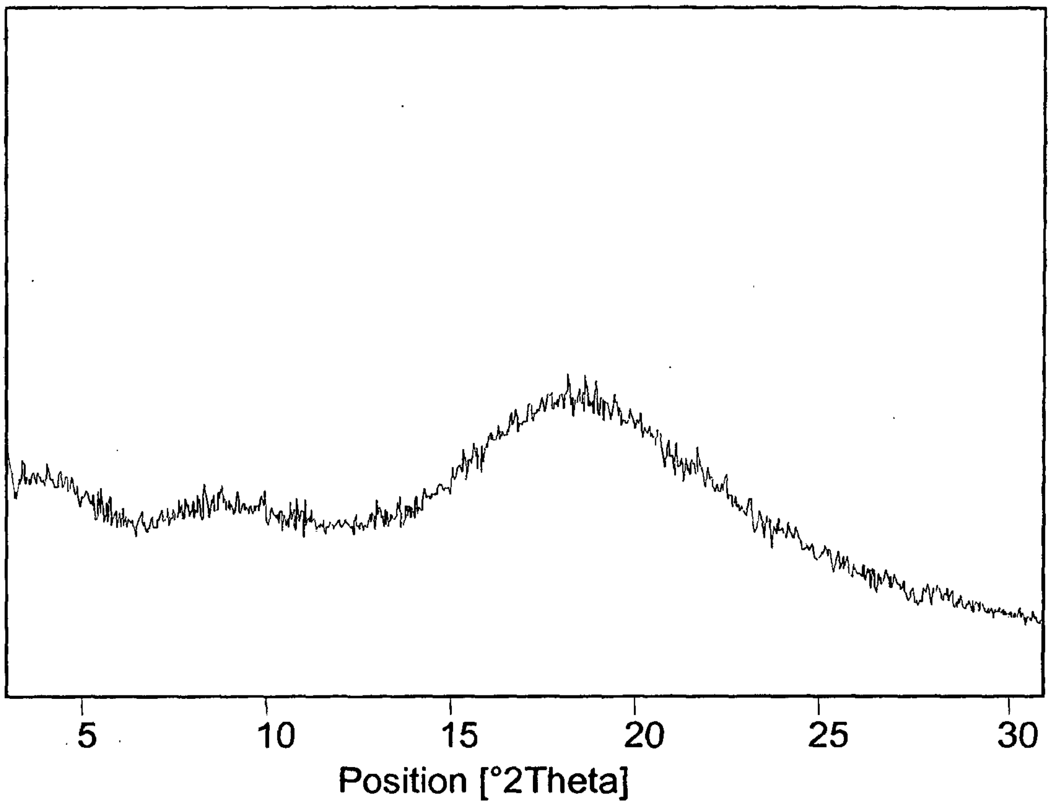

Figure 7

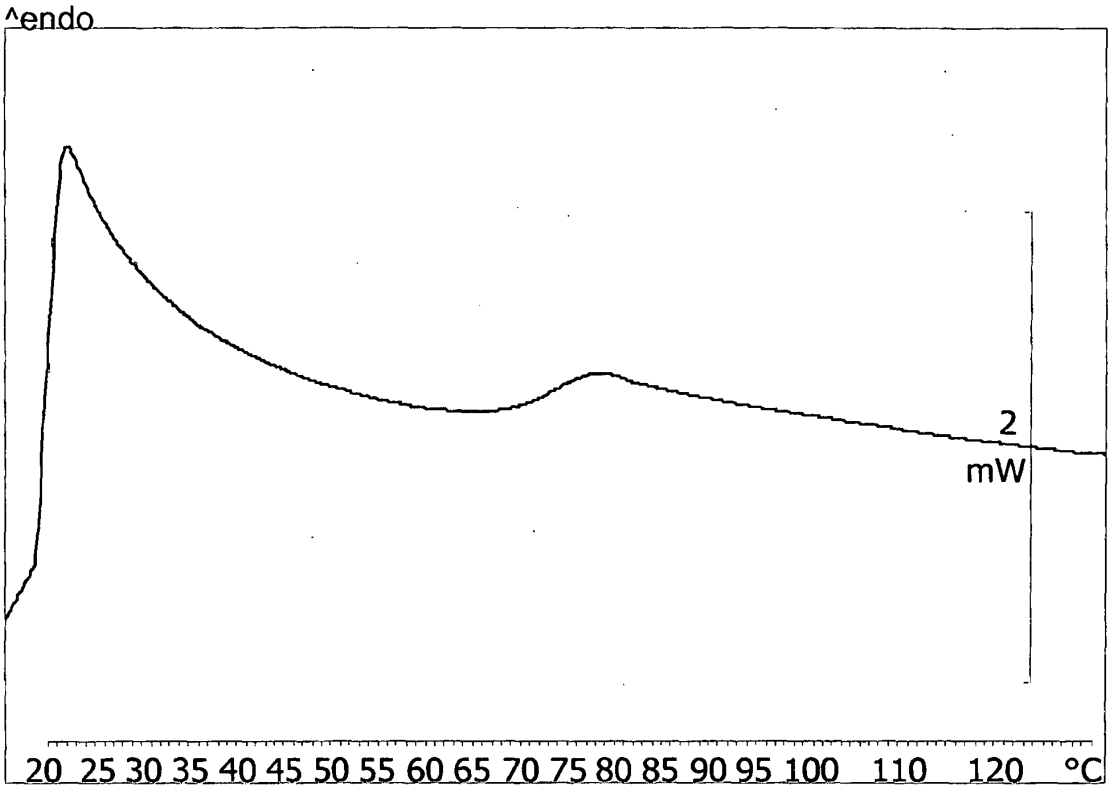

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0678503 A
- EP 1215201 A
- WO 0109079 A
- WO 0109083 A
- WO 0202508 A
- WO 0208172 A
- WO 03103653 A
- WO 2006024501 A
- WO 2006131304 A
- WO 2007039183 A
- GB 2431640 A
- GB 2431641 A
- GB 2431642 A
- GB 2431643 A
- GB 2431644 A
- GB 2431645 A
- GB 2431646 A
- GB 2431647 A
- GB 2431648 A
- GB 2431649 A
- GB 2431650 A
- GB 2431651 A
- GB 2431652 A
- GB 2431653 A
- GB 2431654 A
- WO 2007048620 A
- EP 1517682 A
- WO 2005089729 A
- EP 07006055 A
- EP 1341533 A
- EP 1602370 A
- EP 1507558 A
- US 20030114389 A
- WO 03099279 A
- WO 2005070406 A
- WO 2005077418 A


### Non-patent literature cited in the description


- **KOCIENSKI.** Protecting Groups. Georg Thieme Verlag, 2005
- **GREENE ; WUTS.** Protective Groups in Organic Synthesis. John Wiley & Sons, 1999
- *Tetrahedron,* 1997, vol. 53, 13757